(19) Europäisches Patentamt
European Patent Office
Office européen des brevets

(11) EP 1 290 012 B1

(12) # EUROPEAN PATENT SPECIFICATION

(45) Date of publication and mention of the grant of the patent:
**10.05.2006 Bulletin 2006/19**

(21) Application number: **01943442.2**

(22) Date of filing: **23.05.2001**

(51) Int Cl.:
***C07K 5/068*** *(2006.01)* ***C07K 5/02*** *(2006.01)*
***A61K 38/05*** *(2006.01)* ***A61P 35/00*** *(2006.01)*

(86) International application number:
**PCT/EP2001/005952**

(87) International publication number:
**WO 2001/089282 (29.11.2001 Gazette 2001/48)**

(54) **2-AMINO-3-HYDROXY-4-TERT-LEUCYL-AMINO-5-PHENYL-PENTANOIC ACID AMIDE DERIVATIVES**

AMIDDERIVATE DER 2-AMINO-3-HYDROXY-4-TERT-LEUCYL-AMINO-5-PHENYL-PENTANSÄURE

DERIVES DE L'AMIDE DE L'ACIDE 2-AMINO-3-HYDROXY-4-TERT-LEUCYL-AMINO-5-PHENYL-PENTANOIQUE

(84) Designated Contracting States:
**AT BE CH CY DE DK ES FI FR GB GR IE IT LI LU MC NL PT SE TR**
Designated Extension States:
**RO SI**

(30) Priority: **25.05.2000 GB 0012795**

(43) Date of publication of application:
**12.03.2003 Bulletin 2003/11**

(73) Proprietors:
- **Novartis AG**
  **4056 Basel (CH)**
  Designated Contracting States:
  **BE CH CY DE DK ES FI FR GB GR IE IT LU MC NL PT SE TR**
- **Novartis Pharma GmbH**
  **1230 Wien (AT)**
  Designated Contracting States:
  **AT**

(72) Inventors:
- **FURET, Pascal**
  **F-68800 Thann (FR)**
- **GARCIA-ECHEVERRIA, Carlos**
  **CH-4052 Basel (CH)**
- **IMBACH, Patricia**
  **CH-4303 Kaiseraugst (CH)**
- **LANG, Marc**
  **F-68200 Mulhouse (FR)**
- **ZIMMERMANN, Johann**
  **79424 Auggen (DE)**

(74) Representative: **Gros, Florent et al**
**Novartis AG**
**4002 Basel (CH)**

(56) References cited:
**EP-A- 0 982 317** **WO-A-00/64863**

- **FURET P ET AL.: "Modeling of the Binding Mode of a Non-covalent Inhibitor of the 20S Proteasome. Application to Structure-Based Analogue Design" BIOORGANIC & MEDICAL CHEMISTRY LETTERS, vol. 11, 21 May 2001 (2001-05-21), pages 1321-1324, XP001059888**
- **BILLICH A ET AL.: "Potent and orally bioavailable HIV-1 proteinase inhibitors containing the 2-aminobenzylstatine moiety" ANTIVIRAL CHEMISTRY & CHEMOTHERAPY, vol. 6, no. 5, 1995, pages 327-336, XP001053716**
- **RINGHOFER S ET AL.: "X-ray Structure and Conformational Dynamics of the HIV-1 Protease in Complex with the Inhibitor SDZ283-910: Agreement of Time-resolved Spectroscopy and Molecular Dynamics Simulations" JOURNAL OF MOLECULAR BIOLOGY, vol. 286, 1999, pages 1147-1159, XP000924910**
- **KUNGL AJ ET AL.: "Time-Resolved Fluorescence Anisotropy of HIV-1 Protease Inhibitor Complexes Correlates with Inhibitory Activity" BIOCHEMISTRY, vol. 37, 13 February 1998 (1998-02-13), pages 2778-2786, XP002191310**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- LUM RT: "Selective Inhibition of the Cymotrypsin-Like Activity of the 20S Proteasome by 5-Methoxy-1-Indanone Dipeptide Benzamides" BIOORGANIC & MEDICAL CHEMISTRY LETTERS, vol. 8, 1998, pages 209-214, XP004136850

## Description

The invention relates to 2-amino-3-hydroxy-4-*tert*-leucyl-amino-5-phenyl-pentanoic acid amide derivatives, to processes for the preparation thereof, to medicaments comprising those compounds, and to the use thereof in the preparation of pharmaceutical compositions for the therapeutic treatment of warm-blooded animals, including humans.

The invention relates to compounds of formula I

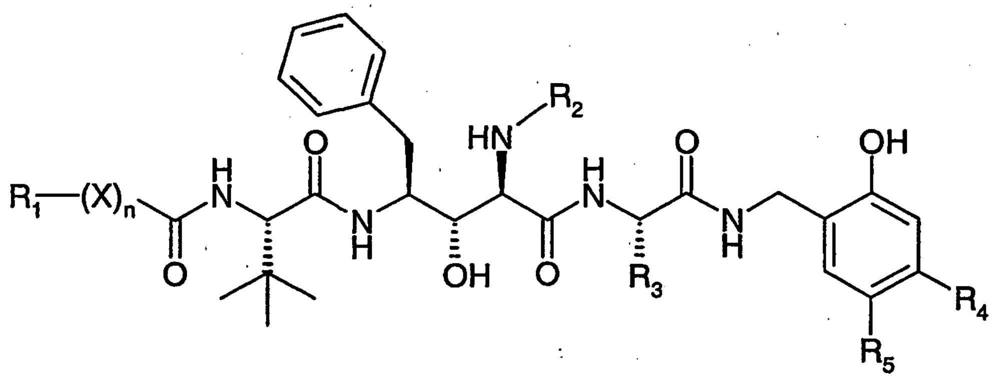

(I),

wherein

n is 0 or 1;

$R_1$ and $R_2$ are independently of the other an aliphatic radical, or an aromatic, aromatic-aliphatic, cycloaliphatic, cycloaliphatic-aliphatic, heterocyclic or heterocyclic-aliphatic radical, each radical having not more than 20 carbon atoms;

$R_3$ is hydrogen, oxa-alkyl, an aliphatic radical or a radical with up to 20 carbon atoms of the formula $-(Y)_m-R_6$, wherein Y is alkyl, m is 0 or 1 and $R_6$ is an unsubstituted or substituted monocyclic radical with 5 or 6 ring members containing up to 3 hetero atoms selected from the group consisting of nitrogen, oxygen and sulfur, wherein said monocyclic radical can also be fused to a lierizo ring;

$R_4$ and $R_5$ are independently selected from the group consisting of hydrogen; an aliphatic radical; free, etherified or esterified hydroxy; free or esterified carboxy; formyl; alkanoyl; unsubstituted, mono- or di-substituted amino; mercapto; sulfo; alkyl-thio; carbamoyl; *N*-alkyl-carbamoyl; *N*,*N*-di-alkyl-carbamoyl; cyano and nitro; wherein carbon containing radicals $R_4$ and $R_5$ have up to 12 carbon atoms and X is nitrogen, oxygen or sulfur; and

if one of the radicals $R_4$ or $R_5$ is hydrogen, the other is as defined above for $R_4$ and $R_5$ with the exception of hydrogen, and if $R_4$ is methoxy, $R_2$ is as defined above with the exception of benzyl monosubstituted at position 4 by halogen, alkyl, alkoxy or hydroxyalkoxy; or a salt thereof.

Within the context of the present Application, the general terms used hereinbefore and hereinafter preferably have the following meanings:

n is 0 or 1, preferably 0.

An aliphatic radical has up to 12 carbon atoms, preferably up to 7 carbon atoms, most preferably up to 4 carbon atoms, and is such an unsubstituted or substituted aliphatic hydrocarbon radical, that is to say such an unsubstituted or substituted alkynyl, alkenyl or preferably alkyl radical, one or more substituents preferably being independently selected from the group consisting of free, etherified or esterified hydroxy; free or esterified carboxy; formyl; alkanoyl; unsubstituted, mono- or di-substituted amino; guanidino; mercapto; sulfo; alkyl-thio; carbamoyl; *N*-alkyl-carbamoyl; *N,N*-di-alkyl-carbamoyl; cyano and nitro.

An aliphatic radical $R_1$ is preferably lower alkyl, such as especially *tert*-butyl.

An aliphatic radical $R_3$ is preferably unsubstituted lower alkyl or lower alkyl substituted by hydroxy, carboxy, amino, carbamoyl, guanldino, mercapto or alkyl-thio, most preferably a side chain of the amino acids alanine, leucine, isoleucine, serine, threonine, cysteine, methionine, asparagine, glutamine, aspartate, glutamate, lysine or arginine, especially valine.

An aliphatic radical $R_4$ is preferably methoxy.

An aromatic radical $R_1$ or $R_2$ has not more than 20 carbon atoms, especially not more than 12 carbon atoms, and is unsubstituted or substituted, preferably in each case unsubstituted or substituted phenyl or naphthyl, especially 1-naphthyl, one or more substituents preferably being independently selected from the group consisting of an aliphatic radical; free, etherified or esterified hydroxy; free or esterified carboxy; formyl; alkanoyl; unsubstituted, mono- or di-substituted amino; mercapto; sulfo; alkyl-thio; carbamoyl; *N*-alkyl-carbamoyl; *N*,*N*-di-alkyl-carbamoyl; cyano and nitro,

more preferably being selected from alkyl, e.g. methyl, ethyl or propyl; alkoxy, e.g. methoxy or ethoxy; di-substituted amino, e.g. dimethylamino; halogen, e.g. chloro or bromo; and halogen-alkyl, e.g. trifluoromethyl.

In an aromatic-aliphatic radical $R_1$ or $R_2$ having not more than 20 carbon atoms the aromatic moiety is as defined above and the aliphatic moiety is preferably lower alkyl, such as especially $C_1$-$C_2$alkyl, which is substituted preferably as defined for the aromatic radical or preferably unsubstituted. An aromatic-aliphatic radical $R_1$ is preferably benzyl or naphthalen-1-ylmethyl. An aromatic-aliphatic radical $R_2$ is preferably benzyl substituted in the benzene moiety by 1-5, preferably by 1-3 methoxy groups; benzyl substituted in the benzene moiety, preferably in position 4, by a dimethyl-amino group; or naphthalen-1-ylmethyl. Most preferably an aromatic-aliphatic radical $R_2$ is 2,3,4- or 3,4,5-trimethoxybenzyl.

A cycloaliphatic radical $R_1$ or $R_2$ has up to 20, especially up to 10 carbon atoms, is mono- or poly-cyclic and is substituted preferably as defined for the aromatic radical or preferably unsubstituted, for example such a cycloalkyl radical, especially such a 5- or 6-membered cycloalkyl radical, such as preferably cyclohexyl.

In a cycloaliphatic-aliphatic radical $R_1$ or $R_2$ having not more than 20 carbon atoms the cycloaliphatic moiety is as defined above and the aliphatic moiety is preferably lower alkyl, such as especially $C_1$-$C_2$alkyl, which is substituted preferably as defined for the aromatic radical or preferably unsubstituted, for example cyclohexyl-methyl.

A heterocyclic radical $R_1$ or $R_2$ contains up to 20 carbon atoms, generally up to 12 carbon atoms, and is substituted preferably as defined for the aromatic radical or unsubstituted and is preferably a saturated or unsaturated monocyclic radical having 5 or 6 ring members and 1 to 3 hetero atoms which are preferably selected from the group consisting of nitrogen, oxygen and sulfur, for example, thienyl or pyridyl, or a bi- or tri-cyclic radical wherein, for example, a benzene radical is fused to the mentioned monocyclic radical, especially, for example, indolyl, such as 5-indolyl, or chinolyl, such as 8-chinolyl.

In a heterocyclic-aliphatic radical $R_1$ or $R_2$ having not more than 20 carbon atoms the heterocyclic moiety is as defined above and the aliphatic moiety is preferably lower alkyl, such as especially $C_1$-$C_2$alkyl, which is substituted preferably as defined for the aromatic radical or preferably unsubstituted. A heterocyclic-aliphatic radical $R_1$ or $R_2$ is for example indolyl-methyl, especially 5-indolyl-methyl, or chinolyl-methyl, especially 8-chinolyl-methyl.

Oxa-alkyl $R_3$ is a radical of the formula $-G(-O-CH_2-CH_2)_t-R_7$, in which G and $R_7$ are alkyl, preferably lower alkyl, and t is 1 to 3, preferably 2, and is especially 2-(1,4-dioxa-hexyl)-ethyl.

In a radical of the formula $-(Y)_m-R_6$ having up to 20 carbon atoms, Y is alkyl, preferably lower alkyl, m is 0 or 1 and the radical $R_6$ is a saturated or unsaturated monocyclic radical having 5 or 6 ring members and up to 3 hetero atoms selected from the group consisting of nitrogen, oxygen and sulfur and alternatively containing a fused benzo ring, such a radical being substituted preferably as defined for the aromatic radical or preferably unsubstituted. A radical $R_6$ is preferably bound to Y via a ring carbon atom and is for example an unsubstituted or substituted member selected from the group consisting of cyclopentyl, cyclohexyl, cyclopentadienyl, phenyl, pyrrolidyl, pyrazolidyl, imidazolidyl, tetrahydrofuryl, piperidyl, piperazinyl, morpholinyl, pyrrolyl, pyrazolyl, imidazolyl, furyl, thienyl, pyridyl, pyrazinyl, pyridazinyl, pyrimidinyl, indenyl, naphthyl, indolyl and chinolyl. Most preferably a radical of the formula $-(Y)_m-R_6$ is piperidyl, especially 4-piperidyl, piperazin-ethyl, especially piperazin-1-ylethyl, morpholinyl-ethyl, especially morpholin-4-ylethyl, pyridyl-methyl, such as 2-, 3- or 4-pyridyl-methyl, or a side chain of the amino acids phenylalanine, tyrosine, tryptophane or histidine.

X is preferably oxygen (-O-).

Alkyl is preferably lower alkyl.

The prefix "lower" denotes a radical having up to and including 7, preferably up to and including 4, carbon atoms.

Lower alkyl is, for example, *n*-propyl, isopropyl, *n*-butyl, isobutyl, *sec*-butyl, *tert*-butyl, *n*-pentyl, isopentyl, neopentyl, *n*-hexyl or *n*-heptyl, preferably isobutyl, *sec*-butyl, *tert*-butyl, isopropyl, ethyl or methyl, most preferably isobutyl, ethyl or methyl.

Etherified hydroxy is, for example, alkoxy, especially lower alkoxy, such as ethoxy or methoxy. Esterified hydroxy is preferably hydroxy esterified by an organic carboxylic acid, such as alkanoic acid, or a mineral acid, such as a hydrohalic adic, for example lower alkanoyloxy or especially halogen, such as iodine or especially fluorine, chlorine or bromine.

Esterified carboxy is, for example, alkoxycarbonyl, especially lower alkoxycarbonyl, such as e.g. methoxycarbonyl.

Alkanoyl is, for example, alkylcarbonyl, especially lower alkylcarbonyl, such as e.g. acetyl.

Mono- or di-substituted amino is, for example, *N*-alkylamino or *N*,*N*-dialkylamino, especially *N*-lower alkylamino or lower *N*,*N*-di-lower alkylamino, such as e.g. *N*-methylamino or *N*,*N*-dimethylamino.

Halogen is fluorine, chlorine, bromine or iodine, preferably fluorine, chlorine or bromine.

The structure of formula I as shown above indicates the absolute configuration.

Salt-forming groups in a compound of formula I are groups or radicals having basic or acidic properties. Compounds having at least one basic group or at least one basic radical, for example a free amino group, a pyrazinyl radical or a pyridyl radical, may form acid addition salts, for example with inorganic acids, such as hydrochloric acid, sulfuric acid or a phosphoric acid, or with suitable organic carboxylic or sulfonic acids, for example aliphatic mono- or di-carboxylic acids, such as trifluoroacetic acid, acetic acid, propionic acid, glycolic acid, succinic acid, maleic acid, fumaric acid,

hydroxymaleic acid, malic acid, tartaric acid, citric acid or oxalic acid, or amino acids such as arginine or lysine, aromatic carboxylic acids, such as benzoic acid, 2-phenoxy-benzoic acid, 2-acetoxy-benzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxyethane-sulfonic acid, or aromatic sulfonic acids, for example benzene-, p-toluene- or naphthalene-2-sulfonic acid. When several basic groups are present mono- or poly-acid addition salts may be formed.

Compounds of formula I having acidic groups, for example a free carboxy group in the radical $R_{10}$, may form metal or ammonium salts, such as alkali metal or alkaline earth metal salts, for example sodium, potassium, magnesium or calcium salts, or ammonium salts with ammonia or suitable organic amines, such as tertiary monoamines, for example triethylamine or tri-(2-hydroxyethyl)-amine, or heterocyclic bases, for example *N*-ethyl-piperidine or *N,N'*-dimethyl-piperazine.

Compounds of formula I having both acidic and basic groups can form intemal salts.

For the purposes of isolation or purification, as well as in the case of compounds that are used further as intermediates, it is also possible to use pharmaceutically unacceptable salts. Only pharmaceutically acceptable, non-toxic salts are used for therapeutic purposes, however, and those salts are therefore preferred.

Owing to the close relationship between the novel compounds in free form and in the form of their salts, including those salts that can be used as intermediates, for example in the purification of the novel compounds or for the identification thereof, hereinbefore and hereinafter any reference to the free compounds should be understood as including the corresponding salts, where appropriate and expedient.

The compounds of formula I have valuable pharmacological properties and can be used, for example, as drugs to treat proliferative diseases.

The compounds of formula I inhibit the proteasome activity. It is known that proteins targeted for the degradation by the multicatalytic proteasome complex have *inter alia* functions in the cell-cycle control (e.g. cyclins, p21, p27) and apoptosis (e.g. p53) (Rolfe, M., Chiu, I.M. and Pagano, M., The ubiquitin-mediated proteolytic pathway as therapeutic area, J. Mol. Med. 75, 1997, 5-17). Inhibitors of the proteasome are therefore suitable for the treatment of proliferative diseases which respond to the inhibition of the proteasome activity. Proliferative diseases like psoriaris and tumors, in particular solid tumors like colon tumor, breast tumor, lung tumor and prostate tumor can be mentioned here.

WO 00/64863 discloses the use of structurally related 2,4-diamino-3-hydroxycarboxylic acid derivatives as proteasome inhibitors which can be used in pharmaceutical compositions for the treatment of animals suffering from proliferative diseases.

Lum RT et al. (1998) Bioorganic & Medicinal Chemistry Letters 8: 209-214 discloses structurally different 20S proteasome inhibitors containing an indanone head group coupled to di- and tripeptides.

Kungl AJ et al. (1998) Biochemistry 37: 2778-2786 discloses structurally related compounds which are useful in the treatment of HIV infection.

Inhibition of 20S Proteasome

The compounds of formula I inhibit the 20S proteasome.

The multicatalytic proteasome complex is responsible for the ATP-dependent proteolysis of most cellular proteins. Although the 20S proteasome contains the proteolytic core, it cannot degrade proteins *in vivo* unless it is complexed with 19S caps, at either end of its structure, which itself contains multiple ATPase activities. This larger structure is known as the 26S proteasome and will rapidly degrade proteins that have been targeted for the degradation by the addition of multiple molecules of the 8.5 kDa polypeptide ubiquitin. As mentioned above, proteins targeted for proteasomal degradation have functions in the cell-cycle. The compounds of formula I are therefore highly suitable for the treatment of diseases which respond to inhibition of the activity of the 20S proteasome, which is the case for the proliferative diseases mentioned above.

The inhibition of the chymotrypsin-like activity of the 20S proteasome can be demonstrated by the following experiment. It is based on the hydrolysis of the fluorogenic peptide Suc-LLVY-AMC (Succinyl-leucine-leucine-valine-tyrosine-7-amino-4-methyl-coumarin), which is cleaved exclusively at the Y-AMC bond by the 20S proteasome. Hydrolysis of this peptide is accompanied by an increase in fluorescence intensity [$\lambda_{ex}$ (excitation wavelength): 355 nm; $\lambda_{em}$ (emission wavelength): 460 nm] due to release of the internally quenched 2-aminobenzoyl fluorescence that accompanies diffusion apart of the hydrolysis product Suc-LLVY.

2 μl of a 1 mM solution of a test compound in DMSO (dimethylsulfoxide, 20 μM final concentration in the well) are preincubated for 60 min at room temperature in black 96-well microtiter plates together with a mixture of 1 μl purified human placenta 20S proteasome (Diabetes Forschungsinstitut, Düsseldorf, Germany; about 100 ng proteasome, depending on the proteasome preparation) and 47 μl buffer-Ca [5 mM $CaCl_2$, 20 mM Tris/HCl (Tris-(hydroxymethyl)-aminomethane hydrochloride) pH 8.0]. 3 μl of a 2.67 mM solution of Suc-LLVY-AMC (Bachem, Switzerland) in DMSO (80 μM final concentration in the well) and 47 μl buffer-Ca are mixed and added. The resulting mixture is incubated for 3 - 24 h

at 37 °C. If desired, proteolysis of the substrate can be stopped by addition of 50 μl stop solution (100 mM monochloroacetic acid, 130 mM NaOH, 100 mM acetic acid, pH = 4.3). The fluorescence is monitored with a FLUOROSKAN ASCENT™ microtiter plate reader.

To each measure series two control experiments have to be carried out:

1.) 0% value: 2 μl DMSO are used in the above described assay instead of 2 μl of a 1 mM solution of a test compound in DMSO and 48 μl buffer-Ca instead of a mixture of 47 μl buffer-Ca and 1 μl purified proteasome.
2.) 100% value: in the above described assay 2 μl DMSO are used instead of 2 μl of a 1 mM solution of the test compound in DMSO.

Calculation

$$\%\ \text{remaining activity} = \frac{(\text{value with compound} - 0\%\ \text{value})}{(100\%\ \text{value} - 0\%\ \text{value})} \bullet 100\%$$

The $IC_{50}$ value is defined as that concentration of a compound at which the remaining activity is 50%. Compounds of formula I exhibit an $IC_{50}$ value for the inhibition of the chymotrypsin-like activity of the 20S proteasome in the range of between 1 nM and 1 μM, especially between 2 nM and 200 nM.

The antiproliferative activity of the compounds of formula I can be demonstrated *in vitro* against e.g. the human breast carcinoma cell line MDA-MB435 (HTB-129) obtained from the American Type Culture Collection (ATCC, Rockville, USA). Routinely the 'CellTiter96™' proliferation assay (Promega, Madison MI) is done following the procedure recommended by the supplier. This assay is performed in 96well plates prepared for tissue culture. Cells are seeded in a density of $2.5x10^4$ per well in 50 μl complete MEM [minimal essential medium] (Gibco-LifeTechnologies) supplemented with 10% fetal calf serum, 100 U/ml PenStrep, 1 mM sodium pyruvate, 4 mM L-Glutamine, 20 mM HEPES and Non Essential Amino Acids. Cells are incubated for 24 hours at 37 °C in humidified atmosphere equilibrated with 5% $CO_2$. Test compounds are added to the cell supernatant as serial dilutions in 50 μl MEM-complete per well. Cells are incubated for at least 48 hours at 37 °C in humidified atmosphere equilibrated with 5% $CO_2$. The Tetrazolium dye is added to the cell supernatant in a volume of 15 μl per well and cell cultures are incubated for 4 hours.

Thereafter, the reaction is stopped by addtion of 100 μl of stopsolution per well and the plates are incubated for one additional hour. The conversion of the tetrazolium dye by metabolically active cells yields soluble formazan. The absorbance of the blue colored cell supernatant is proportional to the amount of viable cells. The absorbance is monitored at the wavelenght of 550 and 630 nm using a microtiter plate reader (Dynatech MR5000). Serial dilutions of compound are prepared by first diluting the 10 mM compound stock solution (in DMSO) to a 60 μM test solution in MEM-complete followed by nine successive 1:3 dilutions in MEM-complete. Wells containing MEM-complete serve as negative control, background (0%). Wells containing cells and MEM / 0.6% DMSO serve as positive control, (100%). Calculation of the % remaining activity is done as described above for the inhibition of the chymotrypsin-like activity of the 20S proteasome. The $IC_{50}$ value is defined as that concentration of a compound at which the remaining activity is 50%.

Compounds of formula I exhibit an $IC_{50}$ value for the antiproliferative activity in the range of between 20 nM and 2 μM, especially between 50 nM and 1 μM.

The antitumoral action of the compounds of formula I can also be demonstrated *in vivo:*

*In vivo* evaluation of antitumor action in nude mice using human tumor xenografts

Female or male BALB/c *nu/nu* (Novartis animal farm, Sisseln, Switzerland or Bomholtgaard, Copenhagen, Denmark) mice with subcutaneously transplanted human tumors are used for the evaluation of the antitumor action of the compounds of formula I against cell lines originating from the four tumor types, breast tumor: MCF-7; lung tumor: NCI H596: colon tumor: HCT 116; and prostate tumor: PC 3.

Materials:

Human colon carcinoma HCT 116 (ATCC CCL 247), human squamous cell lung carcinoma NCI H596 (ATCC HTB 178), estrogen-dependent breast carcinoma MCF-7 (ATCC HTB 22), and the human prostate cancer PC 3 cell line are obtained from the American Type Culture Collection (ATCC, Rockville, USA). The cells are cultured at 37 °C in a 5 % v/v $CO_2$ and 80 % relative humidity atmosphere in the following media: NCI H 596: RPMI 1640, 20 % v/v FBS (fetal

bovine serum), 1% w/v glutamine; HCT-116: McCoy's 5A, 10% v/v FBS, 1% w/v glutamine; PC 3: RPMI 1640, 20% v/v FBS, 1% w/v glutamine; MCF-7, RPMI 1640, 20% v/v FBS, 1 % w/v glutamine. All of these cell lines are adherent and can be released by rinsing with Hank's balanced salt and treatment with 0.25% w/v trypsin. All of these cells are prepared as master cell stocks (in culture media supplemented to contain 20% v/v FBS and 7% v/v DMSO) and stored at -125 °C (liquid nitrogen vapors): Working cell stocks are prepared from the master stocks by thawing and expanding the cells over three passages, which are then distributed to vials and frozen. Viability (trypan blue exclusion test using 0.5% w/v trypan blue) prior to freezing is > 90% for all cell lines.

Establishing solid tumors in nude mice:

Mice are kept under controlled conditions [Optimal Hygienic Conditions, (OHC)] with free access to sterile food and water. Tumors are established after subcutaneous injection of cells (a minimum of 2 x $10^6$ cells in 100 μl PBS (phosphate buffered saline) or medium) in carrier mice (4-8 mice per cell line). Injections are made s.c. in the left flank of the mouse mid-way between the tail and head. The resulting tumors are serially passaged for a minimum of three consecutive transplantations prior to start of treatment. Tumors are transplanted when the tumor reaches a volume of 800 to 1000 $mm^3$.

Transplanting solid tumors in nude mice:

Donor mice are anesthetized (Forene®, Abbott, Switzerland) and killed by cervical dislocation. The skin is disinfected and the tumor removed by dissection. The outer edges of the tumor mass is removed using a scalpel, and the resulting mass is trimmed into pieces of about 3-4 mm in height. Sections of 3-4 $mm^2$ are cut and placed into sterile 0.9% w/v NaCl. Sections of tumor that are necrotic are not used.
Tumor fragments are implanted s.c. into the left flank of the recipient mice. Recipient mice are anesthetized (Forene®, Abbott, Switzerland) and the skin on the entire back and sides of the mice is disinfected. The skin 0.5 to 1 cm above the tail is raised and a single 1 to 1.5 cm incision is made. Tumor sections are pushed into a 13-gauge trocar needle. The trocar needle is pushed into the opening of the skin, and advanced under the skin to a point mid-way between the head and the tail. The tumor fragment is deposited by advancing the trocar. The wound is sutured using one or two metal clips.

In the case of estrogen-dependent breast tumors, estrogen pellets (17b-estradiol, 5 mg/ pellet giving a sustained release over 60 days are obtained from Innovative Research of America, Sarasota, USA), are placed subcutaneously in the other flank.
The tumors are allowed to increase until the size is 100 to 150 $mm^3$ before treatment is begun. The tumors are then measured and placed into groups (normally n = 6 to 8) that are balanced according to the mean size and range of tumor volumes. Groups are randomly assigned to treatment groups.

Preparation and application of test compounds:

Stock solutions of 40 mg/ml of compound are dissolved in 100 % DMSO and stirred at room temperature until a clear solutions is obtained. Prior to each administration, 10 % Tween 80 (FLUKA, Buchs, Switzerland) is added to the stock solution and then diluted 1:20 (v/v) with sterile 0.9 % w/v NaCl or water. Solutions and dilutions are prepared daily prior to application. Applications are given 7 days a week (p.o., i.p., s.c. or i.v.). The volumes of application are: p.o., 25 ml/kg; i.p., 25 ml/kg; s.c., 10 ml/kg; i.v., 10 ml/kg.

Measurement of tumor volumes:

Tumor growth is monitored once, twice or three times weekly (depending on the growth rate of the tumor line) and 24 hours after the last treatment by measuring perpendicular diameters. Calipers capable of determining mm distances are used. Tumor volumes are calculated according to the formula $L \times D^2 \times \pi/6$ (L: length; D: diameter). Antitumor activity is expressed as T/C % (mean increase of tumor volumes of treated animals divided by the mean increase of tumor volumes of control animals multiplied by 100). Tumor regression (%) represents the smallest mean tumor volume compared to the mean tumor volume at the start of treatment. Delta (Δ) tumor volumes compared the change in tumor volume during the duration of the experiment (volume on the last treatment day - volume on the first treatment day). Any animals in which the tumor reaches a size exceeding approximately 1500 to 2000 $mm^3$ are sacrificed.

Additional measurements:

Body weights and survival data are also collected. Delta (Δ) body weights are calculated as an indication of tolerability to treatment (weight on the last treatment day - weight on the first treatment day). Statistically significant body

weight loss, or mortalities, are considered indicators of poor tolerability to treatment. Additionally, mice are monitored once or twice daily for general health.

Statistical analyses:

The basic approach for statistical analyses is to use tests for multiple comparisons to judge the statistical significance of differences between treatment groups, and differences within a group to determine if treatment induced stable disease or tumor regressions. As subcutaneous tumor volumes are not always normally distributed, differences in the subcutaneous tumor volumes between treatment groups is determined using the non-parametric Kruskal-Wallis one way ANOVA test on ranked data and the statistical significance of differences between treatment groups as compared to control groups determined using the Dunnett test. Pair wise comparisons between all groups is performed using the Student-Newman-Keuls (SNK) method. If only two groups are compared, the Rank sum test is used. Animal body weights are normally distributed, and changes in body weights within a group are analyzed by paired t-tests and between group differences are analyzed by a One-Way ANOVA and pair-wise comparisons made using the Tukey test. For all tests the level of significance is set at $p < 0.05$.

Another *in vivo* test to determine the antitumoral action of the compounds of formula I is the following hollow fiber assay:

<u>Hollow Fiber Assay: evaluation of antitumor action in nude mice</u>

In this assay four different human tumors encapsulated in hollow fibers are implantated subcutaneously and/or intraperitonealy into nude mice (athymic female outbred nude mice (Ncr nu/nu)). Animals are then treated with a test compound formulated in an appropriate vehicle, while control animals are treated with the vehicle alone. At the end of the experiment fibers are retrieved, and the number of viable cells is measured using a metabolic assay [MTT, 3-(4,5-dimethylthiazol-2-yl)-2,5-diphenyltetrazolium bromide]. Activity of the test compound is measured by comparing the growth of cells in the experimental animals (T) to the growth of cells in the control animals treated with the vehicle alone (C), and is expressed as %T/C.

Materials:

Human colon carcinomas SW 620 and LS 174T, and breast carcinoma MDA-MB-435S are obtained from the American Type Culture Collection (ATCC, Rockville, USA). The colon carcinoma MIP 101, expressing high levels of pgp-1, was originally established from a patient at the Dana Farber Cancer Institute, Boston, USA. All cell lines are grown according to standard tissue culture techniques in RPMI 1640 containing the following additives: 5% FBS (Fetal Bovine Serum), 5 mg/ml insulin, 5 mg/ml transferrin, 5 ng/ml selenous acid, 1 nM β-estradiol, 1 nM testosterone.

Design of the assay:

The human solid tumor cell lines are encapsulated in PVDF (polyvinylidene fluoride) hollow fibers that are permeable to molecules < 500,000 Daltons and that have an inner diameter of 1 mm (Biopore, Spectrum Medical, CA, USA). After encapsulation and prior to implantation into animals, cells are allowed to attach to the inner surface of the fiber by incubation in the tissue culture media for 24 hours. Four hollow fibers are then implanted into one animal intraperitoneally or subcutaneously. In the experimental setup four to six animals are used per group, and the experiment consists of a minimum of three groups:

1. "Time 0" group
2. Control (placebo) group
3. "Treated" (tested compound) group.

Treatment starts 24 hours after implantation of the fibers. Animals are treated once daily, at days 1, 3, and 5. At the same time when the treatment starts, the animals from "Time 0" group are sacrificed, fibers are retrieved, and the number of viable cells at the beginning of the experiment is determined ($T_0$). At day 7 after the implantation all animals are sacrificed, fibers are retrieved, and the number of viable cells is determined for the Control ($C_v$), and for the "Treated" ($T_v$) groups. %T/C is then calculated according to the formula:

$$\%T/C = (T_v - T_0)/(C_v - T_0) \times 100\%$$

Encapsulation of tumor cells in hollow fibers:
Fibers are cut into desired length and soaked for at least 72 h in 70% Ethanol. Afterwards, fibers and instruments for the encapsulation are sterilized. The human solid tumor cell line grown in tissue culture is trypsinized, suspended in a small amount of tissue culture media and transferred into the fibers using a syringe. The fibers are heat sealed and incubated at 37 °C for 24 h in an atmosphere comprising 5% $CO_2$. The fibers are then implantated subcutaneously and/or intraperitonealy into nude mice.

Determination of viable cells in hollow fibers:
1 g MTT is added to 200 ml PBS (phosphate-buffered saline), stirred for 20 min and filtered (0.22 micron filter). 10 ml of this solution are mixed with 40 ml RPMI 1640 with additives to give the MTT working solution. The retrieved fibers are incubated in 5 ml RPMI 1640 for 30 min at 37 °C in 5% $CO_2$ for stabilization. 0.5 ml of MTT working solution are added to each well of the sample plate. The plates are incubated at 37 °C in 5% $CO_2$ for 4 h. The MTT is aspirated out of each well in the sample plate. 2 ml of 2.5% aqueous protamine sulfate solution is added to each well of the sample plate. The plates are incubated at 4 °C for 24h. The protamine sulfate is aspirated out of each well in the sample plate. 2 ml of protamine sulfate are added to each well and the plates are incubated at 4 °C for further 2-4 h. Each fiber is transferred to a well in a 24 well plate. The fibers are cut so that they lie on the bottom of the wells and dried overnight. 250 μl of DMSO are added to each well. The plates are placed on an orbital shaker for 4 h with a cover to protect the MTT from light. 150 μl of each sample are transferred to the appropriate well in a 96 well plate. The plates are read at 540 nm using DMSO as the blanking well.

The bioavailability after oral administration of compounds of formula I can be shown e.g. in the following test: For peroral administration, a solution of the test substance (25 mg/ml) in a suitable solvent such as Cremophor RH40® / Maisine® / propylene glycol / ethanol (38/32/15/15) is prepared. Female Balb/c mice are fasted for 24 hours prior to the start, and throughout the experiment water is given ad libitum. At various times following drug administration, blood samples are obtained by sacrifycing animals under anaesthesia by cutting the vena jugularis, followed by cervical dislocation. Samples are collected in heparinized tubes (typically 0.4-0.6 ml). For sample analysis solid phase extraction and HPLC are used. Drug concentration in the samples is calculated by least-squares linear regression analysis of the peak area ratio (inhibitor/internal standard) of spiked blood standards versus concentration. From the concentration versus time data, the "Area Under the Curve" (AUC) value is calculated by the trapezoidal rule.

Preference is given to compounds of formula I, wherein
n is 0 or 1;
$R_1$ has not more than 20 carbon atoms and is a member selected from the group consisting of $C_1$-$C_7$ alkyl, cycloalkyl $C_1$-$C_7$ alkyl, phenyl $C_1$-$C_7$ alkyl, naphthyl $C_1$-$C_7$ alkyl, and chinolyl-$C_1$-$C_7$ alkyl, the members being unsubstituted or substituted by one or more radicals independently selected from the group consisting of hydroxy, $C_1$-$C_7$ alkoxy, $C_1$-$C_7$ alkanoyloxy, halogen, carboxy, $C_1$-$C_7$ alkoxycarbonyl, formyl, $C_1$-$C_7$ alkanoyl, amino, *N*-$C_1$-$C_7$ alkylamino, *N,N*-di-$C_1$-$C_7$ alkylamino, mercapto, sulfo, $C_1$-$C_7$ alkyl-thio, carbamoyl, *N*-$C_1$-$C_7$ alkyl-carbamoyl, *N,N*-di $C_1$-$C_7$ alkyl-carbamoyl, cyano, nitro and $C_1$-$C_7$ alkyl that can itself be substituted by said radicals;
$R_2$ has not more than 20 carbon atoms and is a member selected from the group consisting of phenyl-$C_1$-$C_7$ alkyl, naphthyl-$C_1$-$C_7$ alkyl, chinolyl-$C_1$-$C_7$ alkyl, indolyl-$C_1$-$C_7$ alkyl and *N*-$C_1$-$C_7$ alkyl-indolyl-$C_1$-$C_7$ alkyl, the members being unsubstituted or substituted by one or more radicals independently selected from the group consisting of hydroxy, $C_1$-$C_7$ alkoxy, $C_1$-$C_7$ alkanoyloxy, halogen, carboxy, $C_1$-$C_7$ alkoxycarbonyl, formyl, $C_1$-$C_7$ alkanoyl, amino, *N*-$C_1$-$C_7$ alkylamino, *N,N*-di-$C_1$-$C_7$ alkylamino, mercapto, sulfo, $C_1$-$C_7$ alkyl-thio, carbamoyl, *N*-$C_1$-$C_7$ alkyl-carbamoyl, *N,N*-di-$C_1$-$C_7$ alkyl-carbamoyl, cyano, nitro and $C_1$-$C_7$ alkyl that can itself be substituted by said radicals;
$R_3$ is hydrogen, a radical of the formula $-G(-O-CH_2-CH_2)_t-R_7$, in which G and $R_7$ are $C_1$-$C_7$ alkyl and t is 1 to 3, unsubstituted $C_1$-$C_7$ alkyl or $C_1$-$C_7$ alkyl substituted by hydroxy, carboxy, amino, carbamoyl, guanidino, mercapto or $C_1$-$C_7$ alkyl-thio and having not more than 12 carbon atoms, or a radical of the formula $-(Y)_m-R_6$, wherein Y is $C_1$-$C_7$ alkyl, m is 0 or 1 and $R_6$ is phenyl, hydroxy-phenyl, imidazolyl, indolyl, piperidyl, piperazinyl, morpholinyl or pyridyl; $R_4$ and $R_5$ are independently selected from the group consisting of hydrogen, hydroxy, $C_1$-$C_7$ alkoxy, $C_1$-$C_7$ alkanoyloxy, halogen, carboxy, $C_1$-$C_7$ alkoxycarbonyl, formyl, $C_1$-$C_7$ alkanoyl, amino, *N*-$C_1$-$C_7$ alkylamino, *N,N*-di-$C_1$-$C_7$ alkylamino, mercapto, sulfo, $C_1$-$C_7$ alkyl-thio, carbamoyl, *N*-$C_1$-$C_7$ alkyl-carbamoyl, *N,N*-di-$C_1$-$C_7$ alkyl-carbamoyl, cyano, nitro and $C_1$-$C_7$ alkyl that can itself be substituted by said radicals, wherein carbon containing radicals $R_4$ and $R_5$ have up to 12 carbon atoms ; and
X is nitrogen, oxygen or sulfur; and
if one of the radicals $R_4$ or $R_5$ is hydrogen, the other is as defined above for $R_4$ and $R_5$ with the exception of hydrogen, and if $R_4$ is methoxy, $R_2$ is as defined above with the exception of benzyl monosubstituted at position 4 by halogen, alkyl, alkoxy or hydroxyalkoxy;

or a salt thereof.

Preference is given especially to compounds of formula I, wherein

n is 0 or 1;

$R_1$ has not more than 20 carbon atoms and is a member selected from the group consisting of $C_1$-$C_7$ alkyl, cycloalkyl-$C_1$-$C_7$ alkyl, phenyl-$C_1$-$C_7$ alkyl, naphthyl-$C_1$-$C_7$ alkyl, and chinolyl-$C_1$-$C_7$ alkyl, the members being unsubstituted or substituted by one or more radicals independently selected from the group consisting of an aliphatic radical; free, etherified or esterified hydroxy; free or esterified carboxy; formyl; alkanoyl; unsubstituted, mono- or di-substituted amino; mercapto; sulfo; alkyl-thio; carbamoyl; *N*-alkyl-carbamoyl; *N,N*-di-alkyl-carbamoyl; cyano and nitro;

$R_2$ has not more than 20 carbon atoms and is a member selected from the group consisting of phenyl $C_1$-$C_7$ alkyl, naphthyl $C_1$-$C_7$ alkyl, chinolyl-$C_1$-$C_7$ alkyl, indolyl $C_1$-$C_7$ alkyl and *N*-$C_1$-$C_7$ alkyl-indolyl-$C_1$-$C_7$ alkyl, the members being unsubstituted or substituted by one or more radicals independently selected from the group consisting of an aliphatic radical; free, etherified or esterified hydroxy; free or esterified carboxy; formyl; alkanoyl; unsubstituted, mono- or di-substituted amino; mercapto; sulfo; alkyl-thio; carbamoyl; *N*-alkyl-carbamoyl; *N*,*N*-di-alkyl-carbamoyl; cyano and nitro;

$R_3$ is hydrogen, oxa-alkyl, an aliphatic radical or a radical with up to 20 carbon atoms of the formula -$(Y)_m$-$R_6$, wherein Y is alkyl, m is 0 or 1 and $R_6$ is an unsubstituted or substituted monocyclic radical with 5 or 6 ring members containing up to 3 hetero atoms selected from the group consisting of nitrogen, oxygen and sulfur, wherein said monocyclic radical can also be fused to a benzo ring;

$R_4$ and $R_5$ are independently selected from the group consisting of hydrogen; an aliphatic radical; free, etherified or esterified hydroxy; free or esterified carboxy; formyl; alkanoyl; unsubstituted, mono- or di-substituted amino; mercapto; sulfo; alkyl-thlo; carbamoyl; *N*-alkyl-carbamoyl; *N,N*-di-alkyl-carbamoyl; cyano and nitro; wherein carbon containing radicals $R_4$ and $R_5$ have up to 12 carbon atoms ; and

X is oxygen; and

if one of the radicals $R_4$ or $R_5$ is hydrogen, the other is as defined above for $R_4$ and $R_5$ with the exception of hydrogen, and if $R_4$ is methoxy, $R_2$ is as defined above with the exception of benzyl monosubstituted at position 4 by halogen, alkyl, alkoxy or hydroxyalkoxy; or a salt thereof.

Special preference is given to compounds of formula I, wherein

n is 0 or 1;

$R_1$ and $R_2$ are independently of the other an aliphatic radical, or an aromatic, aromatic-aliphatic, cycloaliphatic, cycloaliphatic-aliphatic, heterocyclic or heterocyclic-aliphatic radical, each radical having not more than 20 carbon atoms;

$R_3$ is hydrogen, a radical of the formula -G(-O-$CH_2$-$CH_2)_t$-$R_7$, in which G and $R_7$ are $C_1$-$C_7$ alkyl and t is 1 to 3, unsubstituted $C_1$-$C_7$ alkyl or $C_1$-$C_7$ alkyl substituted by hydroxy, carboxy, amino, carbamoyl, guanidino, mercapto or $C_1$-$C_7$ alkyl-thio and having not more than 12 carbon atoms, or a radical of the formula -$(Y)_m$-$R_6$, wherein Y is $C_1$-$C_7$ alkyl, m is 0 or 1 and $R_8$ is phenyl, hydroxy-phenyl, imidazolyl, indolyl, piperidyl, piperazinyl, morpholinyl or pyridyl; $R_4$ and $R_5$ are independently selected from the group consisting of hydrogen; an aliphatic radical; free, etherified or esterified hydroxy; free or esterified carboxy; formyl; alkanoyl; unsubstituted, mono- or di-substituted amino; mercapto; sulfo; alkyl-thio; carbamoyl; *N*-alkyl-carbamoyl; *N,N*-di-alkyl-carbamoyl; cyano and nitro; wherein carbon containing radicals $R_4$ and $R_5$ have up to 12 carbon atoms ; and

X is oxygen; and

if one of the radicals $R_4$ or $R_5$ is hydrogen, the other is as defined above for $R_4$ and $R_5$ with the exception of hydrogen, and if $R_4$ is methoxy, $R_2$ is as defined above with the exception of benzyl monosubstituted at position 4 by halogen, alkyl, alkoxy or hydroxyalkoxy;

or a salt thereof.

Special preference is given especially to compounds of formula I, wherein

n is 0;

$R_1$ and $R_2$ are independently of the other an aliphatic radical, or an aromatic, aromatic-aliphatic, cydoaliphatic, cycloaliphatic-aliphatic, heterocyclic or heterocyclio-aliphatic radical, each radical having not more than 20 carbon atoms;

$R_3$ is hydrogen, oxa-alkyl, an aliphatic radical or a radical with up to 20 carbon atoms of the formula -$(Y)_m$-$R_6$, wherein Y is alkyl, m is 0 or 1 and $R_6$ is an unsubstituted or substituted monocyclic radical with 5 or 6 ring members containing up to 3 hetero atoms selected from the group consisting of nitrogen, oxygen and sulfur, wherein said monocyclic radical can also be fused to a benzo ring; and

$R_4$ and $R_5$ are independently selected from the group consisting of hydrogen; an aliphatic radical; free, etherified or esterified hydroxy; free or esterified carboxy; formyl; alkanoyl; unsubstituted, mono- or di-substituted amino; mercapto; sulfo; alkyl-thio; carbamoyl; *N*-alkyl-carbamoyl; *N*,*N*-di-alkyl-carbamoyl; cyano and nitro; wherein carbon containing radicals $R_4$ and $R_5$ have up to 12 carbon atoms ; and

if one of the radicals $R_4$ or $R_5$ is hydrogen, the other is as defined above for $R_4$ and $R_5$ with the exception of hydrogen, and if $R_4$ is methoxy, $R_2$ is as defined above with the exception of benzyl monosubstituted at position 4 by halogen, alkyl, alkoxy or hydroxyalkoxy;

or a salt thereof.

Preference is given above all to compounds of formula I, wherein
n is 0 or 1;
$R_1$ is $C_1$-$C_7$ alkyl, phenyl-$C_1$-$C_7$ alkyl or naphthyl $C_1$-$C_7$ alkyl;
$R_2$ is mono-, di-, or tri-$C_1$-$C_7$ alkoxy-phenyl-$C_1$-$C_7$ alkyl, *N,N*-di-$C_1$-$C_7$ alkyl-amino-phenyl-$C_1$-$C_7$ alkyl or naphthyl-$C_1$-$C_7$ alkyl;
$R_3$ is $C_1$-$C_7$ alkyl;
$R_4$ and $R_5$ are independentyl selected from the group consisting of hydrogen, $C_1$-$C_7$ alkoxy or halogen ; and
X is oxygen; and
if one of the radicals $R_4$ or $R_5$ is hydrogen, the other is as defined above for $R_4$ and $R_5$ with the exception of hydrogen, and if $R_4$ is methoxy, $R_2$ is as defined above with the exception of benzyl monosubstituted at position 4 by halogen, alkyl, alkoxy or hydroxyalkoxy;
or a salt thereof.

Most especially preferred are the compounds of formula I described in the Examples and pharmaceutically acceptable salts thereof.

The compounds of formula I or salts thereof are prepared in accordance with processes known per se. The processes according to the invention are as follows:

a) a compound of formula II

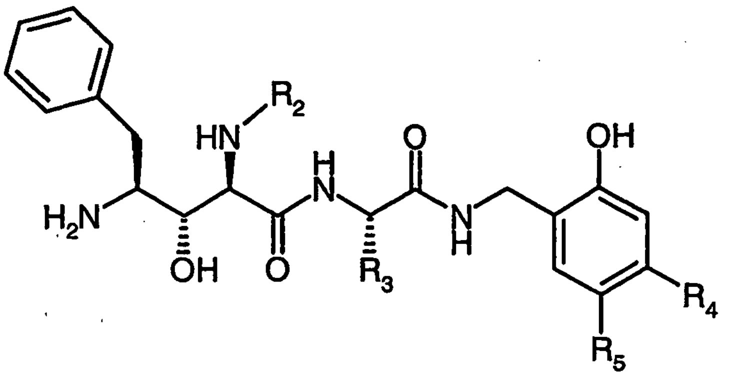

(II),

wherein $R_2$, $R_3$, $R_4$ and $R_5$ are as defined above under formula I, functional groups present in a compound of formula II, with the exception of the groups participating in the reaction, being protected if necessary by readily removable protecting groups, or a salt of such a compound, is reacted with an acid of formula III

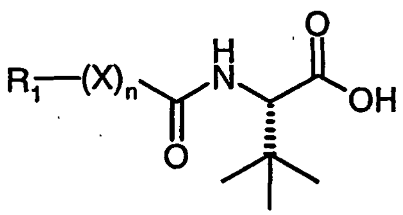

(III),

wherein n, $R_1$ and X are as defined above under formula I, functional groups present in a compound of formula III, with the exception of the groups participating in the reaction, being protected if necessary by readily removable protecting groups, or a suitable reactive acid derivative of such a compound, and any protecting groups present are removed, or

b) a compound of formula IV

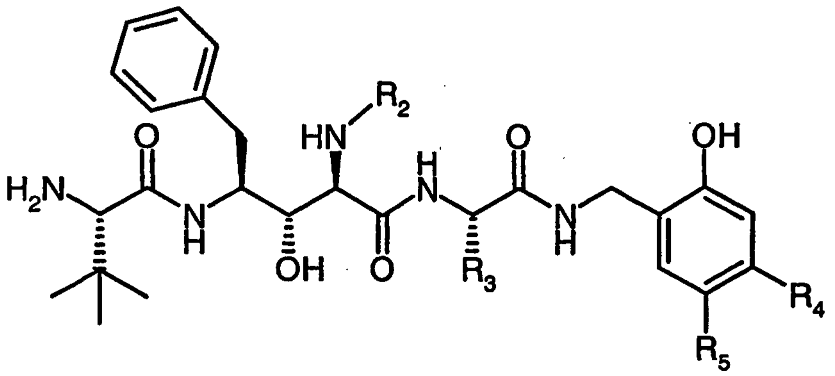

(IV),

wherein $R_2$, $R_3$, $R_4$ and $R_5$ are as defined above under formula I, functional groups present in a compound of formula IV, with the exception of the groups participating in the reaction, being protected if necessary by readily removable protecting groups, or a salt of such a compound, is reacted with an acid of formula V

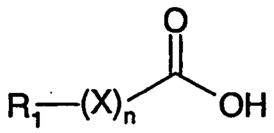

(V),

wherein n, $R_1$ and X are as defined above under formula I, functional groups present in a compound of formula V, with the exception of the groups participating in the reaction, being protected if necessary by readily removable protecting groups, or a suitable reactive acid derivative of such a compound, and any protecting groups present are removed, and, if desired, a compound of formula I obtained by process a) or b) is converted into another compound of formula I, an obtained free compound of formula I is converted into a salt, or an obtained salt of a compound of formula I is converted into another salt or into its free form.

General notes:

The end products of formula I may contain substituents that can also be used as protecting groups in starting materials for the preparation of other end products of formula I. Thus, within the scope of this text, only a readily removable group that is not a constituent of the particular desired end product of formula I is designated a "protecting group", unless the context indicates otherwise.

Protecting groups, and the manner in which they are introduced and removed are described, for example, in "Protective Groups in Organic Chemistry", Plenum Press, London, New York 1973, and in "Methoden der organischen Chemie", Houben-Weyl, 4th edition, Vol. 15/1, Georg-Thieme-Verlag, Stuttgart 1974 and in Theodora W. Greene, "Protective Groups in Organic Synthesis", John Wiley & Sons, New York 1981. A characteristic of protecting groups is that they can be removed readily, i.e. without the occurrence of undesired secondary reactions, for example by solvolysis, reduction, photolysis or alternatively under physiological conditions.

Process a:

The reaction is carried out by dissolving a compound of formula III in a suitable solvent, for example *N,N*-dimethylformamide, *N,N*-dimethylacetamide or *N*-methyl-2-pyrrolidone and by the addition of a suitable base, for example triethylamine, diisopropylethylamine or *N*-methylmorpholine and a suitable coupling agent, for example dicyclohexylcarbodiimide /1-hydroxybenzotriazole (DCC/HOBT); *O*-(1,2-Dihydro-2-oxo-1-pyridyl)-*N,N,N',N'*-tetramethyluronium tetrafluoroborate (TPTU) or 1-(3-dimethylaminopropyl)-3-ethylcarbodiimde hydrochloride (EDC). For review of other coupling agents, see e.g. Klauser; Bodansky, *Synthesis* **1972,** 453-463. The reaction mixture is stirred at a temperature of between approximately -10 °C and room temperature for 0.5 to 1 hour, a compound of formula II is added and stirred

for approximately 16 hours at the same temperature to yield a compound of formula I.

Process b:

The reaction is carried out under the conditions described for process a.
New starting materials and/or transients, as well as processes for the preparation thereof, are likewise the subject of this invention. In the preferred embodiment, such starting materials are used and reaction conditions so selected as to enable the preferred compounds to be obtained.
The starting materials of formulae II - V are known, capable of being prepared according to known processes, or commercially obtainable; in particular, they can be prepared using processes as described in the Examples.
A compound of formula II, wherein the substituents are as defined above under formula I, is obtainable for example by the following reaction sequence:

a compound of formula VI

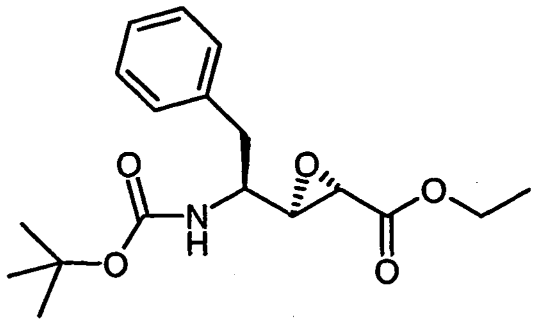

(VI),

is reacted with a compound of formula VII

$H_2N—R_2$ (VII),

wherein $R_2$ is as defined above under formula I. The reaction is carried out by heating the reactants of formulae VI and VII for several hours, for example for from 4 to 6 hours, in the presence of an inert solvent, for example ethanol or isopropanol, at a temperature of approximately 60-90 °C. Hydrolysis of the ethyl ester group of the obtained product yields a compound of formula VIII

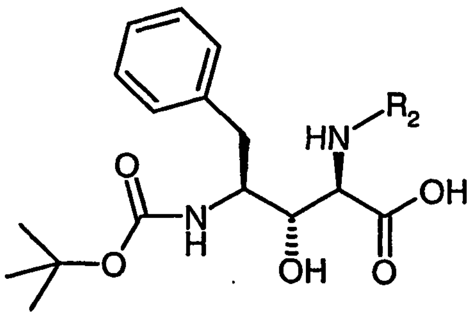

(VIII),

wherein $R_2$ is as defined above under formula I. A compound of formula VIII is then reacted with a compound of formula IX

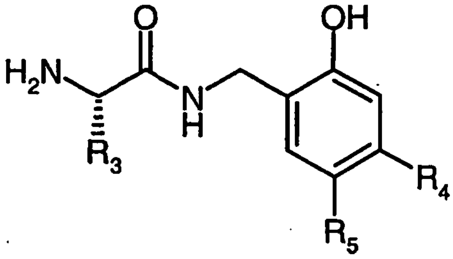

(IX),

wherein the substituents are as defined above under formula I, to obtain a compound of formula X

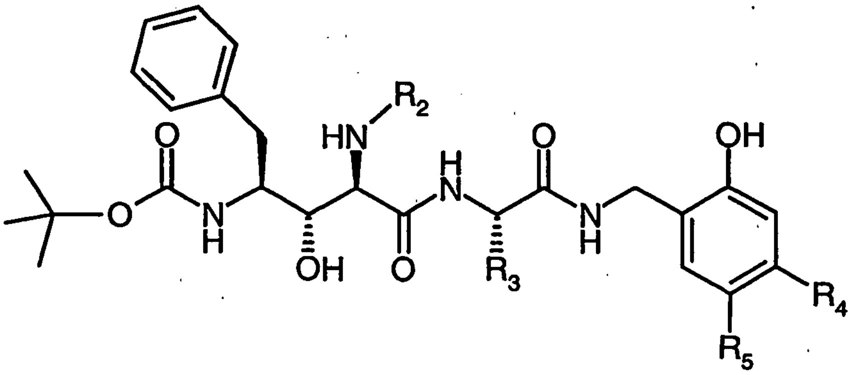

(X),

wherein the substituents are as defined above under formula I. The reaction is carried out as described in process a. From this the corresponding compound of formula II is prepared by removing the *N*-terminal *tert*-butoxycarbonyl protecting group in known manner, for example under the conditions described in Th. W. Greene and P. G. M. Wuts, "Protective Groups in Organic Synthesis", Wiley, New York 1991 (2nd ed., pp. 328-330).

A compound of formula IV, wherein the substituents are as defined above under formula I, is obtainable for example by the following reaction sequence:

a compound of formula II, wherein the substituents are as defined above under formula I, is reacted with a compound of formula XI

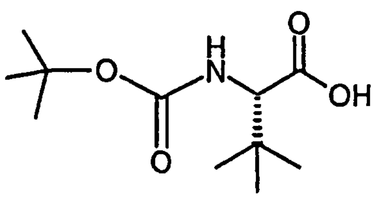

(XI),

to obtain a compound of formula XII

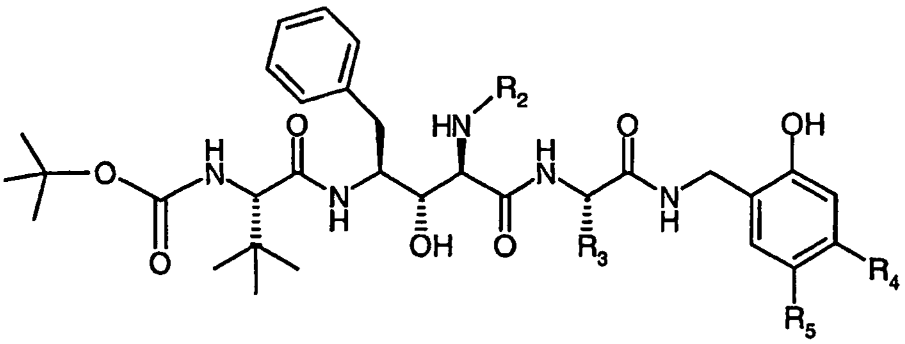

(XII),

wherein the substituents are as defined above under formula I. Compounds of formula XII correspond to compounds of formula I, wherein n is 1, $R_1$ is benzyl and X is oxygen. The reaction is carried out as described in process a. Removal of the *N*-terminal *tert*-butoxycarbonyl protecting group from a compound of formula XII in known manner (see above) yields a compound of formula IV.

The invention relates in particular to the use of a compound of formula I for the preparation of a pharmaceutical composition for treating warm-blooded animals, especially humans, suffering from a proliferative disease, especially a tumor disease and in particular such a disease which responds to inhibition of the multicatalytic proteasome complex Effective doses, for example daily doses of approximately 0.05 g to about 10 g, preferably about 0.1 g to about 5 g, for example about 0.15 g to 1.5 g, of a compound of formula I are administered to a warm-blooded animal of approximately 70 kg body weight according to species, age, individual condition, mode of administration and the individual syndrome.

The invention relates also to pharmaceutical compositions comprising an effective amount, especially an amount effective in the prevention or therapy of one of the above-mentioned diseases, of the active ingredient together with pharmaceutically acceptable carriers that are suitable for topical, enteral, for example oral or rectal, or parenteral administration, and may be inorganic or organic, solid or liquid. For oral administration there are used especially tablets or gelatin capsules comprising the active ingredient together with diluents, for example lactose, dextrose, sucrose, mannitol, sorbitol, cellulose and/or glycerol, and/or lubricants, for example silicic acid, talc, stearic acid or salts thereof, such as magnesium or calcium stearate, and/or polyethylene glycol. Tablets may also comprise binders, for example magnesium aluminium silicate, starches, such as com, wheat or rice starch, gelatin, methylcellulose, sodium carboxymethylcellulose and/or polyvinylpyrrolidone, and, if desired, disintegrators, for example starches, agar, alginic acid or a salt thereof, such as sodium alginate, and/or effervescent mixtures, or adsorbents, dyes, flavourings and sweeteners. The pharmacologically active compounds of the present invention can also be used in the form of parenterally administrable compositions or in the form of infusion solutions. Such solutions are preferably isotonic aqueous solutions or suspensions, which, for example in the case of lyophillsed compositions that comprise the active ingredient alone or together with a carrier, for example mannitol, can be prepared before use. The pharmaceutical compositions may be sterilised and/or may comprise excipients, for example preservatives, stabilisers, wetting agents and/or emulsifiers, solubilisers, salts for regulating the osmotic pressure and/or buffers. The present pharmaceutical compositions which, if desired, may comprise further pharmacologically active substances, such as antibiotics, are prepared in a manner known per se, for example by means of conventional mixing, granulating, confectioning, dissolving or lyophilising processes. The concentrations of active ingredient(s) will, of course, vary depending e.g. on the compound of formula I employed, the treatment desired and the nature of the form. The present pharmaceutical compositions comprise approximately from 1 % to 100 %, especially from approximately 1 % to approximately 20 %, active ingredient(s).

Compositions for oral administration can for example be obtained by formulating a compound of formula I with a carrier medium comprising a hydrophilic phase, a lipophilic phase and a surfactant. Preferably the composition is in the form of a "microemulsion preconcentrate" or "emulsion preconcentrate", in particular of the type providing o/w (oil-in-water) microemulsions or emulsions. However, the composition may be in the form of a microemulsion or an emulsion which additionally contains an aqueous phase, preferably water. A "microemulsion preconcentrate" is a formulation which spontaneously forms a microemulsion in an aqueous medium, for example in water or in the gastric juices after oral application. A microemulsion is a non-opaque or substantially non-opaque colloidal dispersion that is formed spontaneously or substantially spontaneously when its components are brought into contact. A microemulsion is thermodynamically stable. An "emulsion preconcentrate" is a formulation which spontaneously forms an emulsion in an aqueous medium, for example in water or in the gastric juices, after oral application. The emulsion formed is opaque and thermodynamically stable. The lipophilic phase may comprise about 10 to 85 % by weight of the carrier medium; the surfactant may comprise about 5 to 80 % by weight of the carrier medium; and the hydrophilic phase may comprise about 10 to

50 % by weight of the carrier medium.

The hydrophilic phase may be selected from e.g. Transcutol® ($C_2H_5$-[O-($CH_2$)$_2$]$_2$-OH), Glycofurol® (also known as tetrahydrofurfuryl alcohol polyethylene glycol ether) and 1,2-propylene glycol, or mixtures thereof, and is preferably 1,2-propylene glycol. It may include further hydrophilic co-components, for example $C_1$-$C_5$alkanols.

Preferred lipophilic phase components are medium chain fatty acid triglycerides, mixed mono-, di-, tri-glycerides, and transesterified ethoxylated vegetable oils.

Examples of suitable surfactants are:

i) reaction products of a natural or hydrogenated castor oil and ethylene oxide. The oils available under the trade name Cremophor® are especially suitable. Particularly suitable are Cremophor RH 40® and Cremophor RH 60®. Also suitable are polyethyleneglycol castor oils such as that available under the trade name Cremophor EL®. Similar or identical products which may also be used are available under the trade names Nikkol®, Mapeg®, Incrocas® and Tagat®.

ii) Polyoxyethylene-sorbitan-fatty acid esters, for example esters of the type known and commercially available under thetrade name Tween®.

iii) Polyoxyethylene fatty acid esters, for example polyoxyethylene stearic acid esters of the type known and commercially available under the trade name Myrj®.

iv) Polyoxyethylene-polyoxypropylene co-polymers and block co-polymers, e.g. of the type known and commercially available under the trade names Pluronic®, Emkalyx® and Poloxamer®, especially preferred are Pluronic F68® and Poloxamer 188®.

v) Dioctylsulfosuccinate or di-[2-ethylhexyl]-succinate.

vi) Phospliolipids, in particular lecithins.

vii) Propylene glycol mono- and di-fatty acid esters. The surfactant selected preferably has an HLB (hydrophilic/lipophilic balance) of at least 10.

Full physical characteristics of the products referred to herein by trade name can be obtained e.g. from H.P. Fiedler, "Lexikon der Hilfsstoffe für Pharmazie, Kosmetik und Angrenzende Gebiete", Editio Cantor, D-7960 Aulendorf, Germany, 3rd revised and expanded edition (1989).

The pharmaceutical compositions may also include further additives or ingredients, for example antioxidants. They exhibit especially advantageous properties when administered orally, for example in terms of consistency and high level of bioavailability obtained in standard bioavailability trials. Pharmacokinetic parameters, for example absorption and blood levels, also become surprisingly more predictable and problems in administration with erratic absorption may be eliminated or reduced. Additionally, the pharmaceutical composition is effective with tenside materials, for example bile salts, present in the gastro-intestinal tract.

The pharmaceutical compositions for oral use are preferably compounded in unit dosage form, for example by filling them into orally administrable capsule shells. The capsule shells may be soft or hard gelatine capsule shells. However, if desired the pharmaceutical compositions may be in a drink solution form and may include water or any other aqueous system, to provide emulsion or microemulsion systems suitable for drinking.

The present invention relates in particular to the use of compounds of formula I in the manufacture of a pharmaceutical composition for the treatment of a proliferative disease, e.g. of a solid tumor, and their use for the treatment of such a proliferative disease.

The following Examples illustrate the invention but do not limit the invention in any way.

<u>Abbreviations:</u>

| | |
|---|---|
| brine | saturated sodium chloride solution |
| DIEA | *N*-Ethyldiisopropylamine |
| DMF | *N,N*-Dimethylformamide |
| d.t. | decomposition temperature |
| equiv. | equivalent(s) |
| ES-MS | electron spray mass spectrometry |
| h | hour(s) |
| Me | methyl |
| min | minute(s) |
| m.p. | melting point |
| temp. | temperature |

| | |
|---|---|
| THF | tetrahydrofuran |
| TFA | trifluoroacetic acid |
| TPTU | *O*-(1,2-Dihydro-2-oxo-1-pyridyl)-*N,N,N',N'*-tetramethyluronium tetrafluoroborate |
| $t_R$ | retention time |

Example 1: ((S)-1-{(1S,2R,3R)-1-Benzyl-2-hydroxy-3-[(S)-1-(2-hydroxy-4-methoxybenzylcarbamoyl)-2-methyl-propylcarbamoyl]-3-[(naphthalen-1-ylmethyl)-amino]-propylcarbamoyl}-2,2-dimethyl-propyl)-carbamic acid benzyl ester

*N*-Benzyloxycarbonyl-L-*tert*-leucine (1.05 equiv.) is dissolved in *N,N*-dimethylacetamide, and diisopropylethylamine (3.1 equiv.) and TPTU (1.05 equiv.) are added. The resulting mixture is stirred at room temperature for 0.5 - 1 h and then (2R,3R,4S)-4-amino-3-hydroxy-2-[(naphthalen-1-ylmethyl)-amino]-5-phenyl-pentanoic acid [(S)-1-(2-hydroxy-4-methoxybenzylcarbamoyl)-2-methyl-propyl]-amide (1.0 equiv.) is added. After 16 h at room temperature, ethyl acetate is added, the mixture is washed with a 5% $NaHCO_3$ solution and brine. The organic layer is dried, the solvent is evaporated and the residue is chromatographed on silicagel (solvent: dichloromethane-methanol). The title compound is obtained: ES-MS: 847.0; single peak at $t_R$= 7.73 min (System E).

The starting material is obtained as follows:

Step 1.1: To the solution of 3.02 g (9 mmol) of (2S,3R)-3-((S)-1-*tert*-butoxycarbonylamino-2-phenyl-ethyl)-oxirane-2-carboxylic acid ethyl ester* in 23 ml of ethanol 2.6 ml (18 mmol) of 1-naphthyl-methylamine is added and stirred for 4.75 h at 70-80 °C. After removal of the solvent under reduced presssure, the crude product is purified by means of column chromatography on silicagel (solvent: hexanes-ethylacetate 3:1) to yield (2R,3R,4S)-4-*tert*-butoxycarbonylamino-3-hydroxy-2-[(naphthalen-1-ylmethyl)-amino]-5-phenyl-pentanoic acid ethyl ester; ES-MS: 493.0, single peak at $t_R$= 11.18 min (System A).

* The synthesis of this compound is described by Dieter Scholz et al. in *Joumal of Medicinal Chemistry* **1994,** Vol. **37,** No.19, pp. 3079-3089.

Step 1.2: To the solution of 3.20 g (6.5 mmol) (2R,3R,4S)-4-*tert*-butoxycarbonylamino-3-hydroxy-2-[(naphthalen-1-ylmethyl)-amino]-5-phenyl-pentanoic acid ethyl ester in 30 ml of absolute THF is added 7.8 ml of 1 M LiOH solution within 10 min and stirred for 5 h at room temperature. After completion, the reaction mixture was adjusted to pH 4.0 with 1 N HCl. The precipitated product was filtered, washed repeatedly with water and dried to obtain (2R,3R,4S)-4-*tert*-butoxycarbonylamino-3-hydroxy-2-[(naphthalen-1-ylmethyl)-amino]-5-phenyl-pentanoic acid; m.p. 225-227 °C, single peak at $t_R$= 9.68 min (System A).

Step 1.3: To the suspension of 558 mg (1.2 mmol) of (2R,3R,4S)-4-*tert*-butoxycarbonylamino-3-hydroxy-2-[(naphthalen-1-ylmethyl)-amino]-5-phenyl-pentanoic acid in 10 ml of absolute DMF at room temperature is added 392 mg (1.32 mmol) of TPTU (Aldrich 36,572-6) and 0.82 ml (4.8 mmol) of DIEA (Fluka 03340). After 1 h 416 mg (1.44 mmol) of (S)-2-amino-*N*-(2-hydroxy-4-methoxy-benzyl)-3-methyl-butyramide** is added at 0 °C and the reaction mixture is stirred for 1 h at 0-5 °C, then 1 h at room temperature. After completion, the mixture is poured onto ice water, extracted into ethyl acetate and the organic layer is subsequently being washed with water and saline. After filtration from $MgSO_4$ and evaporation the crude product was purified by means of column chromatography on silicagel (solvent: hexanes-ethylacetate 1:2) to yield {(1S,2R,3R)-1-benzyl-2-hydroxy-3-[(S)-1-(2-hydroxy-4-methoxy-benzylcarbamoyl)-2-methylpropylcarbamoyl]-3-[(naphthalen-1-ylmethyl)-amino]-propyl}-carbamic acid *tert*-butyl ester; m.p. 95-98 °C, single peak at $t_R$= 11.54 min (System A).

** The synthesis of this compound is described by A. Billich et al. in *Antiviral Chemistry & Chemotherapy* **1995**, Vol. **6**, No.5, pp. 327-336.

Step 1.4: (2R,3R,4S)-4-Amino-3-hydroxy-2-[(naphthalen-1-ylmethyl)-amino]-5-phenyl-pentanoic acid [(S)-1-(2-hydroxy-4-methoxy-benzylcarbamoyl)-2-methyl-propyl]-amide The N-terminal *tert*-butoxycarbonyl protecting group is removed by treatment of {(1S,2R,3R)-1-benzyl-2-hydroxy-3-[(S)-1-(2-hydroxy-4-methoxy-benzylcarbamoyl)-2-methyl-propylcarbamoyl]-3-[(naphthalen-1-ylmethyl)-amino]-propyl}-carbamic acid *tert*-butyl ester with a 4 N solution of HCl in dioxane using a protocol known in the art. After evaporation of the solvent, the crude compound is taken up in ethyl acetate and washed several times with 5% $NaHCO_3$ and then brine. The organic phase is dried over $MgSO_4$ and the solvent is evaporated to dryness. The compound is used without further purification; ES-MS: 600.1; single peak at $t_R$= 7.31 min (System G).

Example 2: ((S)-1-{(1S,2R,3R)-1-Benzyl-3-(4-dimethylamino-benzylamino)-2-hydroxy-3-[(S)-1-(2-hydroxy-4-methoxy-benzylcarbamoyl)-2-methyl-propylcarbamoyl]-propylcarbamoyl}-2,2-dimethyl-propyl)-carbamic acid benzyl ester

The title compound is obtained starting from *N*-benzyloxycarbonyl-L-*tert*-leucine and (2R,3R,4S)-4-amino-2-(4-dimethylamino-benzylamino)-3-hydroxy-5-phenyl-pentanoic acid [(S)-1-(2-hydroxy-4-methoxy-benzylcarbamoyl)-2-methyl-propyl]-amide as described in Example 1; ES-MS: 840.0, single peak at $t_R$= 6.87 min (System E).
The starting material is obtained as follows:

Step 2.1: (2R,3R,4S)-4-*tert*-Butoxycarbonylamino-2-(4-dimethylamino-benzylamino)-3-hydroxy-5-phenyl-pentanoic acid ethyl ester is prepared as described in Example 1, Step 1.1, but using 4-*N*-dimethyl-benzylamine as a reagent; ES-MS: 486.0, single peak at $t_R$= 8.35 min (System A).

Step 2.2: (2R,3R,4S)-4-*tert*-Butoxycarbonylamino-2-(4-dimethylamino-benzylamino)-3-hydroxy-5-phenyl-pentanoic acid is prepared as described in Example 1, Step 1.2, but using (2R,3R,4S)-4-*tert*-butoxy-carbonylamino-2-(4-dimethylamino-benzylamino)-3-hydroxy-5-phenyl-pentanoic acid ethyl ester as the starting material; m.p. 202-204 °C, single peak at $t_R$= 6.90 min (System A).

Step 2.3: {(1S,2R,3R)-1-Benzyl-3-(4-dimethylamino-benzylamino)-2-hydroxy-3-[(S)-1-(2-hydroxy-4-methoxy-benzylcarbamoyl)-2-methyl-propylcarbamoyl]-propyl}-carbamic acid *tert*-butyl ester is prepared from (2R,3R,4S)-4-*tert*-butoxycarbonylamino-2-(4-dimethylamino-benzylamino)-3-hydroxy-5-phenyl-pentanoic acid and (S)-2-Amino-*N*-(2-hydroxy-4-methoxybenzyl)-3-methyl-butyramide as described in Example 1, Step 1.3; m.p. 89-91 °C, single peak at $t_R$= 10.21 min (System A).

Step 2.4: (2R,3R,4S)-4-Amino-2-(4-dimethylamino-benzylamino)-3-hydroxy-5-phenyl-pentanoic acid [(S)-1-(2-hydroxy-4-methoxy-benzylcarbamoyl)-2-methyl-propyl]-amide The title compound is obtained from {(1S,2R,3R)-1-benzyl-3-(4-dimethylamino-benzylamino)-2-hydroxy-3-[(S)-1-(2-hydroxy-4-methoxy-benzylcarbamoyl)-2-methyl-propylcarbamoyl]-propyl}-carbamic acid *tert*-butyl ester as described in Example 1, Step 1.4; ES-MS: 593.0; single peak at $t_R$= 6.03 min (System G).

Example 3: {(S)-1-[(1S,2R,3R)-1-Benzyl-3-[(S)-1-(5-bromo-2-hydroxy-benzyl-carbamoyl)-2-methyl-propylcarbamoyl]-2-hydroxy-3-(4-methoxy-benzylamino)-propylcarbamoyl]-2,2-dimethyl-propyl}-carbamic acid benzyl ester

The title compound is obtained from *N*-benzyloxycarbonyl-L-*tert*-leucine and (2R,3R,4S)-4-amino-3-hydroxy-2-(4-methoxy-benzylamino)-5-phenyl-pentanoic acid [(S)-1-(5-bromo-2-hydroxy-benzylcarbamoyl)-2-methyl-propyl]-amide as described in Example 1; ES-MS: 875.9, single peak at $t_R$= 6.74 min (System D).

Example 4: {(S)-1-[(1S,2R,3R)-1-Benzyl-2-hydroxy-3-[(S)-1-(2-hydroxy-4-methoxy-benzylcarbamoyl)-2-methyl-propylcarbamoyl]-3-(3-methoxy-benzylamino)-propylcarbamoyl]-2,2-dimethyl-propyl}-carbamic acid benzyl ester

The title compound is obtained from *N*-benzyloxycarbonyl-L-*tert*-leucine and (2R,3R,4S)-4-amino-3-hydroxy-2-(3-methoxy-benzylamino)-5-phenyl-pentanoic acid [(S)-1-(2-hydroxy-4-methoxy-benzylcarbamoyl)-2-methyl-propyl]-amide as described in Example 1; ES-MS: 827.1, single peak at $t_R$= 8.35 min (System E).
The starting material is obtained as follows:

Step 4.1: (2R,3R,4S)-4-*tert*-Butoxycarbonylamino-3-hydroxy-2-(3-methoxy-benzylamino)-5-phenyl-pentanoic acid ethyl ester is prepared as described in Example 1, Step 1.1, but using 3-methoxy-benzylamine as a reagent; ES-MS: 473, single peak at $t_R$= 5.49 min (System C).

Step 4.2: (2R,3R,4S)-4-*tert*-Butoxycarbonylamino-3-hydroxy-2-(3-methoxy-benzylamino)-5-phenyl-pentanoic acid is prepared as described in Example 1, Step 1.2, but using (2R,3R,4S)-4-*tert*-butoxycarbonylamino-3-hydroxy-2-(3-methoxy-benzylamino)-5-phenyl-pentanoic acid ethyl ester as the starting material; d.t. 210 °C, single peak at $t_R$=4.80 min (System C).

Step 4.3: [(1S,2R,3R)-1-Benzyl-2-hydroxy-3-[(S)-1-(2-hydroxy-4-methoxy-benzylcarbamoyl)-2-methyl-propylcarbamoyl]-3-(3-methoxy-benzylamino)-propyl]-carbamic acid *tert*-butyl ester is prepared from (2R,3R,4S)-4-*tert*-butoxycarbonylamino-3-hydroxy-2-(3-methoxy-benzylamino)-5-phenyl-pentanoic acid and (S)-2-Amino-*N*-(2-hydroxy-4-methoxy-benzyl)-3-methyl-butyramide as described in Example 1, Step 1.3; ES-MS: 679.0, single peak at $t_R$= 5.89 min (System C).

Step 4.4: (2R,3R,4S)-4-Amino-3-hydroxy-2-(3-methoxy-benzylamino)-5-phenyl-pentanoic acid [(S)-1-(2-hydroxy-4-methoxy-benzylcarbamoyl)-2-methyl-propyl]-amide The title compound is obtained from [(1S,2R,3R)-1-benzyl-2-hydroxy-3-[(S)-1-(2-hydroxy-4-methoxy-benzylcarbamoyl)-2-methyl-propylcarbamoyl]-3-(3-methoxy-benzylamino)-propyocarbamic acid *tert*-butyl ester as described in Example 1; ES-MS: 580:1; single peak at $t_R$= 6.69 min (System G).

Example 5: ((S)-1-{(1S,2R,3R)-1-Benzyl-3-(2,4-dimethoxy-benzylamino)-2-hydroxy-3-[(S)-1-(2-hydroxy-4-methoxy-benzylcarbamoyl)-2-methyl-propylcarbamoyl]-propylcarbamoyl}-2,2-dimethyl-propyl)-carbamic acid benzyl ester

The title compound is obtained from *N*-benzyloxycarbonyl-L-*tert*-leucine and (2R,3R,4S)-4-amino-2-(2,4-dimethoxy-benzylamino)-3-hydroxy-5-phenyl-pentanoic acid [(S)-1-(2-hydroxy-4-methoxy-benzylcarbamoyl)-2-methyl-propyl]-amide as described in Example 1; ES-MS: 857.1, single peak at $t_R$= 7.46 min (System E).

The starting material is obtained as follows:

Step 5.1: (2R,3R,4S)-4-*tert*-Butoxycarbonylamino-2-(2,4-dimethoxy-benzylamino)-3-hydroxy-5-phenyl-pentanoic acid ethyl ester is prepared as described in Example 1, Step 1.1, but using 2,4,-dimethoxy-benzylamine as a reagent; ES-MS: 503.0, single peak at $t_R$= 5.57 min (System C).

Step 5.2: (2R,3R,4S)-4-*tert*-Butoxycarbonylamino-2-(2,4-dimethoxy-benzylamino)-3-hydroxy-5-phenyl-pentanoic acid is prepared as described in Example 1, Step 1.2, but using (2R,3R,4S)-4-*tert*-butoxycarbonylamino-2-(2,4-dimethoxy-benzylamino)-3-hydroxy-5-phenyl-pentanoic acid ethyl ester as the starting material; d.t. 221 °C, single peak at $t_R$= 4.92 min (System C).

Step 5.3: {(1S,2R,3R)-1-Benzyl-3-(2,4-dimethoxy-benzylamino)-2-hydroxy-3-[(S)-1-(2-hydroxy-4-methoxy-benzylcarbamoyl)-2-methyl-propylcarbamoyl]-propyl}-carbamic acid *tert*-butyl ester is prepared from (2R,3R,4S)-4-*tert*-Butoxycarbonylamino-2-(2,4-dimethoxy-benzylamino)-3-hydroxy-5-phenyl-pentanoic acid and (S)-2-amino-*N*-(2-hydroxy-4-methoxybenzyl)-3-methyl-butyramide as described in Example 1, Step 1.3; ES-MS: 709.0, single peak at $t_R$= 5.89 min (System C).

Step 5.4: (2R,3R,4S)-4-Amino-2-(2,4-dimethoxy-benzylamino)-3-hydroxy-5-phenyl-pentanoic acid [(S)-1-(2-hydroxy-4-methoxy-benzylcarbamoyl)-2-methyl-propyl]-amide The title compound is obtained from {(1S,2R,3R)-1-benzyl-3-(2,4-dimethoxy-benzylamino)-2-hydroxy-3-[(S)-1-(2-hydroxy-4-methoxy-benzylcarbamoyl)-2-methyl-propylcarbamoyl]-propyl}-carbamic acid *tert*-butyl ester as described in Example 1; ES-MS: 610.1; single peak at $t_R$= 7.45 min (System G).

Example 6: {(S)-1-[(1S,2R,3R)-1-Benzyl-2-hydroxy-3-[(S)-1-(2-hydroxy-4-methoxy-benzylcarbamoyl)-2-methyl-propylcarbamoyl]-3-(3,4,5-trimethoxy-benzylamino)-propylcarbamoyl]-2,2-dimethyl-propyl}-carbamic acid benzyl ester

The title compound is obtained from *N*-benzyloxycarbonyl-L-*tert*-leucine and (2R,3R,4S)-4-amino-3-hydroxy-5-phenyl-2-(3,4,5-trimethoxy-benzylamino)-pentanoic acid [(S)-1-(2-hydroxy-4-methoxy-benzylcarbamoyl)-2-methyl-propyl]-amide as described in Example 1. Purification of the crude compound is performed by semi-preparative HPLC. The purified compound is taken up in ethyl acetate and washed several times with 5% $NaHCO_3$ and then brine. The organic phase is dried over $MgSO_4$ and the solvent is evaporated to dryness; ES-MS: 887.0, single peak at $t_R$= 7.41 min (System F).

The starting material is obtained as follows:

Step 6.1: (2R,3R,4S)-4-*tert*-Butoxycarbonylamino-3-hydroxy-5-phenyl-2-(3,4,5-trimethoxy-benzylamino)-pentanoic acid ethyl ester is prepared as described in Example 1, Step 1.1, but using 3,4,5-trimethoxybenzylamine as a reagent; ES-MS: 533.0, single peak at $t_R$= 5.34 min (System C).

Step 6.2: (2R,3R,4S)-4-*tert*-Butoxycarbonylamino-3-hydroxy-5-phenyl-2-(3,4,5-trimethoxy-benzylamino)-pentanoic acid is prepared as described in Example 1, Step 1.2, but using (2R,3R,4S)-4-*tert*-Butoxycarbonylamino-3-hydroxy-5-phenyl-2-(3,4,5-trimethoxy-benzylamino)-pentanoic acid ethyl ester as the starting material; d.t. 206.8 °C, single peak at $t_R$= 4.66 min (System C).

Step 6.3: {(1S,2R,3R)-1-Benzyl-3-(3,4,5-trimethoxy-benzylamino)-2-hydroxy-3-[(S)-1-(2-hydroxy-4-methoxy-benzylcarbamoyl)-2-methyl-propylcarbamoyl]-propyl}-carbamic acid *tert*-butyl ester is prepared from (2R,3R,4S)-4-*tert*-butoxycarbonylamino-2-(3,4,5-trimethoxy-benzylamino)-3-hydroxy-5-phenyl-pentanoic acid and (S)-2-amino-*N*-(2-hydroxy-4-methoxybenzyl)-3-methyl-butyramide as described in Example 1, Step 1.3; ES-MS: 739.0, single peak at $t_R$= 5.69 min (System C).

Step 6.4: (2R,3R,4S)-4-Amino-3-hydroxy-5-phenyl-2-(3,4,5-trimethoxy-benzylamino)-pentanoic acid [(S)-1-(2-hydroxy-4-methoxy-benzylcarbamoyl)-2-methyl-propyl]-amide The title compound is obtained from [(1S,2R,3R)-1-benzyl-2-hydroxy-3-[(S)-1-(2-hydroxy-4-methoxy-benzylcarbamoyl)-2-methyl-propylcarbamoyl]-3-(3,4,5-trimethoxy-benzylamino)-propyl]-carbamic acid *tert*-butyl ester as described in Example 1; ES-MS: 640.2; single peak at $t_R$= 6.44 min (System H).

Example 7: {(S)-1-[(1S,2R,3R)-1-Benzyl-2-hydroxy-3-[(S)-1-(2-hydroxy-4-methoxy-benzylcarbamoyl)-2-methyl-propylcarbamoyl]-3-(2,3,4-trimethoxy-benzylamino)-propylcarbamoyl]-2,2-dimethyl-propyl}-carbamic acid benzyl ester

The title compound is obtained from *N*-benzyloxycarbonyl-L-*tert*-leucine and (2R,3R,4S)-4-amino-3-hydroxy-5-phenyl-2-(2,3,4-trimethoxy-benzylamino)-pentanoic acid [(S)-1-(2-hydroxy-4-methoxy-benzylcarbamoyl)-2-methyl-propyl]-amide as described in Example 1. The crude compound is purified by semi-preparative HPLC. The purified compound is taken up in ethyl acetate and washed several times with 5% $NaHCO_3$ and then brine. The organic phase is dried over $MgSO_4$ and the solvent is evaporated to dryness; ES-MS: 887.0, single peak at $t_R$= 7.18 min (System E).

The starting material is obtained as follows:

Step 7.1: (2R,3R,4S)-4-*tert*-Butoxycarbonylamino-3-hydroxy-5-phenyl-2-(2,3,4-trimethoxy-benzylamino)-pentanoic acid ethyl ester is prepared as described in Example 1, Step 1.1, but using 2,3,4-trimethoxybenzylamine as a reagent; ES-MS: 533.0, single peak at $t_R$= 13.17 min (System B).

Step 7.2: (2R,3R,4S)-4-*tert*-Butoxycarbonylamino-3-hydroxy-5-phenyl-2-(2,3,4-trimethoxy-benzylamino)-pentanoic acid is prepared as described in Example 1, Step 1.2, but using (2R,3R,4S)-4-*tert*-butoxycarbonylamino-3-hydroxy-5-phenyl-2-(2,3,4-trimethoxy-benzylamino)-pentanoic acid ethyl ester as the starting material; d.t. 207 °C, single peak at $t_R$= 11.69 min (System B).

Step 7.3: {(1S,2R,3R)-1-Benzyl-3-(2,3,4-trimethoxy-benzylamino)-2-hydroxy-3-[(S)-1-(2-hydroxy-4-methoxy-benzylcarbamoyl)-2-methyl-propylcarbamoyl]-propyl}-carbamic acid *tert*-butyl ester is prepared from (2R,3S,4S)-4-*tert*-butoxycarbonylamino-2-(2,3,4-trimethoxy-benzylamino)-3-hydroxy-5-phenyl-pentanoic acid and (S)-2-amino-*N*-(2-hydroxy-4-methoxybenzyl)-3-methyl-butyramide as described in Example 1, Step 1.3; ES-MS: 739.0, single peak at $t_R$= 5.90 min (System C).

Step 7.4: (2R,3R,4S)-4-Amino-3-hydroxy-5-phenyl-2-(2,3,4-trimethoxy-benzylamino)-pentanoic acid [(S)-1-(2-hydroxy-4-methoxy-benzylcarbamoyl)-2-methyl-propyl]-amide The title compound is obtained from [(1S,2R,3R)-1-benzyl-2-hydroxy-3-[(S)-1-(2-hydroxy-4-methoxy-benzylcarbamoyl)-2-methyl-propylcarbamoyl]-3-(2,3,4-trimethoxy-benzylamino)-propyl]-carbamic acid *tert*-butyl ester as described in Example 1; ES-MS: 640.1; single peak at $t_R$= 6.53 min (System H).

Example 8: ((S)-1-{(1S,2R,3R)-1-Benzyl-3-(3,4-dimethoxy-benzylamino)-2-hydroxy-3-[(S)-1-(2-hydroxy-4-methoxy-benzylcarbamoyl)-2-methyl-propylcarbamoyl]-propylcarbamoyl}-2,2-dimethyl-propyl)-carbamic acid benzyl ester

The title compound is obtained from *N*-benzyloxycarbonyl-L-*tert*-leucine and (2R,3R,4S)-4-amino-2-(3,4-dimethoxy-benzylamino)-3-hydroxy-5-phenyl-pentanoic acid [(S)-1-(2-hydroxy-4-methoxy-benzylcarbamoyl)-2-methyl-propyl]-amide as described in Example 1; ES-MS: 857.1, single peak at $t_R$= 8.25 min (System E).

The starting material is obtained as follows:

Step 8.1: (2R,3R,4S)-4-*tert*-Butoxycarbonylamino-3-hydroxy-5-phenyl-2-(3,4-dimethoxy-benzylamino)-pentanoic acid ethyl ester is prepared as described in Example 1, Step 1.1, but using 3,4-dimethoxybenzylamine as a reagent; ES-MS: 503.0, single peak at $t_R$= 5.22 min (System C).

Step 8.2: (2R,3R,4S)-4-*tert*-Butoxycarbonylamino-3-hydroxy-5-phenyl-2-(3,4-dimethoxy-benzylamino)-pentanoic acid is prepared as described in Example 1, Step 1.2, but using (2R,3R,4S)-4-*tert*-butoxycarbonylamino-3-hydroxy-5-phenyl-2-(3,4-dimethoxy-benzylamino)-pentanoic acid ethyl ester as the starting material; d.t. 222 °C, single peak at $t_R$= 4.54 min (System C).

Step 8.3: {(1S,2R,3R)-1-Benzyl-3-(3,4-dimethoxy-benzyl amino)-2-hydroxy-3-[(S)-1-(2-hydroxy-4-methoxy-benzyl-

carbamoyl)-2-methyl-propylcarbamoyl]-propyl}-carbamic acid *tert*-butyl ester is prepared from (2R,3R,4S)-4-*tert*-butoxycarbonylamino-2-(3,4-dimethoxy-benzylamino)-3-hydroxy-5-phenyl-pentanoic acid and (S)-2-amino-*N*-(2-hydroxy-4-methoxybenzyl)-3-methyl-butyramide as described in Example 1, Step 1.3; m.p. 143.6 °C, single peak at $t_R$= 5.57 min (System C).

Step 8.4: (2R,3R,4S)-4-Amino-2-(3,4-dimethoxy-benzylamino)-3-hydroxy-5-phenyl-pentanoic acid [(S)-1-(2-hydroxy-4-methoxy-benzylcarbamoyl)-2-methyl-propyl]-amide The title compound is obtained from {(1S,2R,3R)-1-benzyl-3-(3,4-dimethoxy-benzylamino)-2-hydroxy-3-[(S)-1-(2-hydroxy-4-methoxy-benzylcarbamoyl)-2-methyl-propylcarbamoyl]-propyl}-carbamic acid *tert*-butyl ester as described in Example 1; ES-MS: 610.1; single peak at $t_R$= 6.61 min (System G).

Example 9: ((S)-1-{(1S,2R,3R)-1-Benzyl-3-(3,5-dimethoxy-benzylamino)-2-hydroxy-3-[(S)-1-(2-hydroxy-4-methoxy-benzylcarbamoyl)-2-methyl-propylcarbamoyl]-propylcarbamoyl}-2,2-dimethyl-propyl)-carbamic acid benzyl ester

The title compound is obtained from *N*-benzyloxycarbonyl-L-*tert*-leucine and (2R,3R,4S)-4-amino-2-(3,5-dimethoxy-benzylamino)-3-hydroxy-5-phenyl-pentanoic acid [(S)-1-(2-hydroxy-4-methoxy-benzylcarbamoyl)-2-methyl-propyl]-amide as described in Example 1; ES-MS: 857.1, single peak at $t_R$= 7.61 min (System E).
The starting material is obtained as follows:

Step 9.1: (2R,3R,4S)-4-*tert*-Butoxycarbonylamino-3-hydroxy-5-phenyl-2-(3,5-dimethoxy-benzylamino)-pentanoic acid ethyl ester is prepared as described in Example 1, Step 1.1, but using 3,5-dimethoxybenzylamine as a reagent; ES-MS: 503.0, single peak at $t_R$= 5.82 min (System C).

Step 9.2: (2R,3R,4S)-4-*tert*-Butoxycarbonylamino-3-hydroxy-5-phenyl-2-(3,4-dimethoxy-benzylamino)-pentanoic acid is prepared as described in Example 1, Step 1.2, but using (2R,3R,4S)-4-*tert*-butoxycarbonylamino-3-hydroxy-5-phenyl-2-(3,5-dimethoxy-benzylamino)-pentanoic acid ethyl ester as the starting material; d.t. 206 °C, single peak at $t_R$= 4.92 min (System C).

Step 9.3: {(1S,2R,3R)-1-Benzyl-3-(3,5-dimethoxy-benzyl amino)-2-hydroxy-3-[(S)-1-(2-hydroxy-4-methoxy-benzylcarbamoyl)-2-methyl-propylcarbamoyl]-propyl}-carbamic acid *tert*-butyl ester is prepared from (2R,3R,4S)-4-*tert*-butoxycarbonylamino-2-(3,5-dimethoxy-benzylamino)-3-hydroxy-5-phenyl-pentanoic acid and (S)-2-amino-*N*-(2-hydroxy-4-methoxybenzyl)-3-methyl-butyramide as described in Example 1, Step 1.3; m.p. 138.2 °C, single peak at $t_R$= 5.87 min (System C).

Step 9.4: (2R,3R,4S)-4-Amino-2-(3,5-dimethoxy-benzylamino)-3-hydroxy-5-phenyl-pentanoic acid [(S)-1-(2-hydroxy-4-methoxy-benzylcarbamoyl)-2-methyl-propyl]-amide

The title compound is obtained from {(1S,2R,3R)-1-benzyl-3-(3,5-dimethoxy-benzylamino)-2-hydroxy-3-[(S)-1-(2-hydroxy-4-methoxy-benzylcarbamoyl)-2-methyl-propylcarbamoyl]-propyl}-carbamic acid *tert*-butyl ester as described in Example 1; ES-MS: 609.1; single peak at $t_R$= 7.07 min (System G).

Example 10: {(S)-1-[(1S,2R,3R)-1-Benzyl-2-hydroxy-3-[(S)-1-(2-hydroxy-4-methoxy-benzylcarbamoyl)-2-methyl-propylcarbamoyl]-3-(2,4,5-trimethoxy-benzylamino)-propylcarbamoyl]-2,2-dimethyl-propyl}-carbamic acid benzyl ester

The title compound is obtained from *N*-benzyloxycarbonyl-L-*tert*-leucine and (2R,3R,4S)-4-amino-3-hydroxy-5-phenyl-2-(2,4,5-trimethoxy-benzylamino)-pentanoic acid [(S)-1-(2-hydroxy-4-methoxy-benzylcarbamoyl)-2-methyl-propyl]-amide as described in Example 1; ES-MS: 887.0, single peak at $t_R$= 7.28 min (System E).
The starting material is obtained as follows:

Step 10.1: (2R,3R,4S)-4-*tert*-Butoxycarbonylamino-3-hydroxy-5-phenyl-2-(2,4,5-trimethoxy-benzylamino)-pentanoic acid ethyl ester is prepared as described in Example 1, Step 1.1, but using 2,4,5-trimethoxybenzylamine as a reagent; ES-MS: 533.0, single peak at $t_R$= 5.33 min (System C).

Step 10.2: (2R,3R,4S)-4-*tert*-Butoxycarbonylamino-3-hydroxy-5-phenyl-2-(2,4,5-trimethoxy-benzylamino)-pentanoic acid is prepared as described in Example 1, Step 1.2, but using (2R,3R,4S)-4-*tert*-butoxycarbonylamino-3-hydroxy-5-phenyl-2-(2,4,5-trimethoxy-benzylamino)-pentanoic acid ethyl ester as the starting material; m.p. 206 °C, single peak at $t_R$=11.25 min (System B).

Step 10.3: {(1S,2R,3R)-1-Benzyl-3-(2,4,5-trimethoxy-benzyl amino)-2-hydroxy-3-[(S)-1-(2-hydroxy-4-methoxy-benzylcarbamoyl)-2-methyl-propylcarbamoyl]-propyl}-carbamic acid *tert*-butyl ester is prepared from (2R,3R,4S)-4-*tert*-butoxycarbonylamino-2-(2,4,5-trimethoxy-benzylamino)-3-hydroxy-5-phenyl-pentanoic acid and (S)-2-amino-*N*-(2-hydroxy-4-methoxybenzyl)-3-methyl-butyramide as described in Example 1, Step 1.3; ES-MS: 739.0, single peak at $t_R$= 5.66 min (System C).

Step 10.4: (2R,3R,4S)-4-Amino-3-hydroxy-5-phenyl-2-(2,4,5-trimethoxy-benzyl-amino)-pentanoic acid [(S)-1-(2-hydroxy-4-methoxy-benzylcarbamoyl)-2-methyl-propyl]-amide The title compound is obtained from [(1S,2R,3R)-1-benzyl-2-hydroxy-3-[(S)-1-(2-hydroxy-4-methoxy-benzylcarbamoyl)-2-methyl-propylcarbamoyl]-3-(2,4,5-trimethoxy-benzylamino)-propyl]-carbamic acid *tert*-butyl ester as described in Example 1; ES-MS: 639.1; single peak at $t_R$= 7.33 min (System G).

Example 11: {(S)-1-[(1S,2R,3R)-1-Benzyl-2-hydroxy-3-[(S)-1-(2-hydroxy-4-methoxy-benzylcarbamoyl)-2-methyl-propylcarbamoyl]-3-(3,4,5-trimethoxy-benzylamino)-propylcarbamoyl]-2,2-dimethyl-propyl}-carbamic acid *tert*-butyl ester

The title compound is obtained starting from *N*-*tert*-butoxycarbonyl-L-*tert*-leucine and (2R,3R,4S)-4-Amino-3-hydroxy-5-phenyl-2-(3,4,5-trimethoxy-benzylamino)-pentanoic acid [(S)-1-(2-hydroxy-4-methoxy-benzylcarbamoyl)-2-methyl-propyl]-amide (described in Example 6), and using the coupling conditions described in Example 1. The crude compound is purified by semi-preparative HPLC. The purified compound is taken up in ethyl acetate and washed several times with 5% $NaHCO_3$ and then brine. The organic phase is dried over $MgSO_4$ and the solvent is evaporated to dryness; ES-MS: 853.1, single peak at $t_R$= 7.28 min (System F).

Example 12: (2R,3R,4S)-4-[(S)-3,3-Dimethyl-2-(2-naphthalen-1-yl-acetylamino)-butyrylamino]-3-hydroxy-5-phenyl-2-(3,4,5-trimethoxy-benzylamino)-pentanoic acid [(S)-1-(2-hydroxy-4-methoxy-benzylcarbamyl)-2-methyl-propyl]-amide

The title compound is obtained starting from 1-naphthylacetic acid (Fluka, Buchs, Switzerland) and (2R,3R,4S)-4-((S)-2-amino-3,3-dimethyl-butyrylamino)-3-hydroxy-5-phenyl-2-(3,4,5-trimethoxy-benzylamino)-pentanoic acid [(S)-1-(2-hydroxy-4-methoxy-benzylcarbamoyl)-2-methyl-propyl]-amide, and using the coupling conditions described in Example 1. The crude compound is purified by semi-preparative HPLC. The purified compound is taken up in ethyl acetate and washed several times with 5% $NaHCO_3$ and then brine. The organic phase is dried over $MgSO_4$ and the solvent is evaporated to dryness; ES-MS: 921.0, single peak at $t_R$= 7.56 min (System F).

The starting material is obtained as follows:

Step 12.1: (2R,3R,4S)-4-((S)-2-Amino-3,3-dimethyl-butyrylamino)-3-hydroxy-5-phenyl-2-(3,4,5-trimethoxy-benzylamino)-pentanoic acid [(S)-1-(2-hydroxy-4-methoxy-benzylcarbamoyl)-2-methyl-propyl]-amide
The title compound is obtained starting from {(S)-1-[(1S,2R,3R)-1-benzyl-2-hydroxy-3-[(S)-1-(2-hydroxy-4-methoxy-benzylcarbamoyl)-2-methyl-propylcarbamoyl]-3-(3,4,5-trimethoxy-benzylamino)-propylcarbamoyl]-2,2-dimethyl-propyl}-carbamic acid *tert*-butyl ester (Example 11) as described in Example 1; ES-MS: 753.1; single peak at $t_R$= 7.56 min (System F).

Example 13: (2R,3R,4S)-4-[(S)-3,3-Dimethyl-2-(2-naphthalen-1-yl-acetylamino)-butyrylamino]-3-hydroxy-5-phenyl-2-(2,3,4-trimethoxy-benzylamino)-pentanoic acid [(S)-1-(2-hydroxy-4-methoxy-benzylcarbamoyl)-2-methyl-propyl]-amide

The title compound is obtained starting from 1-naphthylacetic acid (Fluka, Buchs, Switzerland) and (2R,3R,4S)-4-((S)-2-amino-3,3-dimethyl-butyrylamino)-3-hydroxy-5-phenyl-2-(2,3,4-trimethoxy-benzylamino)-pentanoic acid [(S)-1-(2-hydroxy-4-methoxy-benzylcarbamoyl)-2-methyl-propyl]-amide, and using the coupling conditions described in Example 1. The crude compound is purified by semi-preparative HPLC. The purified compound is taken up in ethyl acetate and washed several times with 5% $NaHCO_3$ and then brine. The organic phase is dried over $MgSO_4$ and the solvent is evaporated to dryness; ES-MS: 921.0, single peak at $t_R$= 7.71 min (System F).

The starting material is obtained as follows:

Step 13.1: {(S)-1-[(1S,2R,3R)-1-Benzyl-2-hydroxy-3-[(S)-1-(2-hydroxy-4-methoxy-benzylcarbamoyl)-2-methyl-propylcarbamoyl]-3-(2,3,4-trimethoxy-benzylamino)-propylcarbamoyl]-2,2-dimethyl-propyl}-carbamic acid *tert*-butyl ester The title compound is obtained starting from *N*-*tert*-butoxycarbonyl-L-*tert*-leucine and (2R,3R,4S)-4-amino-3-hydroxy-5-phenyl-2-(2,3,4-trimethoxy-benzylamino)-pentanoic acid [(S)-1-(2-hydroxy-4-methoxy-benzylcarbamoyl)-2-methyl-propyl]-amide (described in Example 7), and using the coupling conditions described in Example

1. The crude compound is purified by semi-preparative HPLC. The purified compound is taken up in ethyl acetate and washed several times with 5% $NaHCO_3$ and then brine. The organic phase is dried over $MgSO_4$ and the solvent is evaporated to dryness; ES-MS: 853.1, single peak at $t_R$= 7.34 min (System F).

Step 13.2: (2R,3R,4S)-4-((S)-2-Amino-3,3-dimethyl-butyrylamino)-3-hydroxy-5-phenyl-2-(2,3,4-trimethoxy-benzylamino)-pentanoic acid [(S)-1-(2-hydroxy-4-methoxy-benzylcarbamoyl)-2-methyl-propyl]-amide

The title compound is obtained starting from {(S)-1-[(1S,2R,3R)-1-benzyl-2-hydroxy-3-[(S)-1-(2-hydroxy-4-methoxy-benzylcarbamoyl)-2-methyl-propylcarbamoyl]-3-(2,3,4-trimethoxy-benzylamino)-propylcarbamoyl]-2,2-dimethyl-propyl}-carbamic acid *tert*-butyl ester as described in Example 1; ES-MS: 753.1; single peak at $t_R$= 5.58 min (System F).

Example 14: (2R,3R,4S)-4-[(S)-3,3-Dimethyl-2-(2-naphthalen-1-yl-acetylamino)-butyrylamino]-3-hydroxy-5-phenyl-2-(2,4,5-trimethoxy-benzylamino)-pentanoic acid [(S)-1-(2-hydroxy-4-methoxy-benzylcarbamoyl)-2-methyl-propyl]-amide

The title compound is obtained from 1-naphthylacetic acid (Fluka, Buchs, Switzerland) and (2R,3R,4S)-4-((S)-2-amino-3,3-dimethyl-butyrylamino)-3-hydroxy-5-phenyl-2-(2,4,5-trimethoxy-benzylamino)-pentanoic acid [(S)-1-(2-hydroxy-4-methoxy-benzylcarbamoyl)-2-methyl-propyl]-amide, and using the coupling conditions described in Example 1; ES-MS: 921.1, single peak at $t_R$= 7.47 min (System E).

The starting material is obtained as follows:

Step 14.1: {(S)-1-[(1S,2R,3R)-1-Benzyl-2-hydroxy-3-[(S)-1-(2-hydroxy-4-methoxy-benzylcarbamoyl)-2-methyl-propylcarbamoyl]-3-(2,4,5-trimethoxy-benzylamino)-propylcarbamoyl]-2,2-dimethyl-propyl}-carbamic acid *tert*-butyl ester The title compound is obtained starting from *N*-*tert*-butoxycarbonyl-L-*tert*-leucine and (2R,3R,4S)-4-amino-3-hydroxy-5-phenyl-2-(2,4,5-trimethoxy-benzylamino)-pentanoic acid [(S)-1-(2-hydroxy-4-methoxy-benzylcarbamoyl)-2-methyl-propyl]-amide (described in Example 10), and using coupling conditions described in Example 1; ES-MS: 853.1, single peak at $t_R$= 7.29 min (System E).

Step 14.2: (2R,3R,4S)-4-((S)-2-Amino-3,3-dimethyl-butyrylamino)-3-hydroxy-5-phenyl-2-(2,4,5-trimethoxy-benzylamino)-pentanoic acid [(S)-1-(2-hydroxy-4-methoxy-benzylcarbamoyl)-2-methyl-propyl]-amide

The title compound is obtained staring from {(S)-1-[(1S,2R,3R)-1-benzyl-2-hydroxy-3-[(S)-1-(2-hydroxy-4-methoxy-benzylcarbamoyl)-2-methyl-propylcarbamoyl]-3-(2,4,5-trimethoxy-benzylamino)-propylcarbamoyl]-2,2-dimethyl-propyl}-carbamic acid *tert*-butyl ester as described in Example 1; ES-MS: 753.1 single peak at $t_R$= 5.71 min (System E).

Example 15: (2R,3R,4S)-4-[(S)-3,3-Dimethyl-2-(2-naphthalen-1-yl-acetylamino)-butyrylamino]-3-hydroxy-2-[(naphthalen-1-ylmethyl)-amino]-5-phenyl-pentanoic acid [(S)-1-(2-hydroxy-4-methoxy-benzylcarbamoyl)-2-methyl-propyl]-amide

The title compound is obtained starting from 1-naphthylacetic acid (Fluka, Buchs, Switzerland) and (2R,3R,4S)-4-((S)-2-amino-3,3-dimethyl-butyrylamino)-3-hydroxy-2-[(naphthalen-1-ylmethyl)-amino]-5-phenyl-pentanoic acid [(S)-1-(2-hydroxy-4-methoxy-benzylcarbamoyl)-2-methyl-propyl]-amide, and using the coupling conditions described in Example 1; ES-MS: 880.5, single peak at $t_R$= 8.83 min (System E).

The starting material is obtained as follows:

Step 15.1: ((S)-1-{(1S,2R,3R)-1-Benzyl-2-hydroxy-3-[(S)-1-(2-hydroxy-4-methoxy-benzylcarbamoyl)-2-methyl-propylcarbamoyl]-3-[(naphthalen-1-ylmethyl)-amino]-propylcarbamoyl}-2,2-dimethyl-propyl)-carbamic acid *tert*-butyl ester The title compound is obtained starting from *N*-*tert*-butoxycarbonyl-L-*tert*-leucine and (2R,3R,4S)-4-amino-3-hydroxy-2-[(naphthalen-1-ylmethyl)-amino]-5-phenyl-pentanoic acid [(S)-1-(2-hydroxy-4-methoxy-benzylcarbamoyl)-2-methyl-propyl]-amide (described in Example 1), and using the coupling conditions described in Example 1; ES-MS: 813.4, single peak at $t_R$= 8.94 min (System E).

Step 15.2: (2R,3R,4S)-4-((S)-2-Amino-3,3-dimethyl-butyrylamino)-3-hydroxy-2-[(naphthalen-1-ylmethyl)-amino]-5-phenyl-pentanoic acid [(S)-1-(2-hydroxy-4-methoxy-benzylcarbamoyl)-2-methyl-propyl]-amide

The title compound is obtained starting from ((S)-1-{(1S,2R,3R)-1-benzyl-2-hydroxy-3-[(S)-1-(2-hydroxy-4-methoxy-benzylcarbamoyl)-2-methyl-propylcarbamoyl]-3-[(naphthalen-1-ylmethyl)-amino]-propylcarbamoyl}-2,2-dimethyl-propyl)-carbamic acid *tert*-butyl ester as described in Example 1; ES-MS: 713.4; single peak at $t_R$= 7.56 min (System E).

Example 16: (2R,3R,4S)-2-(4-Dimethylamino-benzylamino)-4-[(S)-3,3-dimethyl-2-(2-naphthalen-1-yl-acetylamino)-butyrylamino]-3-hydroxy-5-phenyl-pentanoic acid [(S)-1-(2-hydroxy-4-methoxy-benzylcarbamoyl)-2-methyl-propyl]-amide.

The title compound is obtained starting from 1-naphthylacetic acid (Fluka, Buchs, Switzerland) and (2R,3R,4S)-4-((S)-2-amino-3,3-dimethyl-butyrylamino)-2-(4-dimethylamino-benzylamino)-3-hydroxy-5-phenyl-pentanoic acid [(S)-1-(2-hydroxy-4-methoxy-benzylcarbamoyl)-2-methyl-propyl]-amide, and using the coupling conditions described in Example 1; ES-MS: 874.5, single peak at $t_R$= 8.23 min (System E).

The starting material is obtained as follows:

Step 16.1: ((S)-1-{(1S,2R,3R)-1-Benzyl-3-(4-dimethylamino-benzylamino)-2-hydroxy-3-[(S)-1-(2-hydroxy-4-methoxy-benzylcarbamoyl)-2-methyl-propylcarbamoyl]-propylcarbamoyl}-2,2-dimethyl-propyl)-carbamic acid *tert*-butyl ester The title compound is obtained starting from *N-tert*-butoxycarbonyl-L-*tert*-leucine and (2R,3R,4S)-4-amino-2-(4-dimethylamino-benzylamino)-3-hydroxy-5-phenyl-pentanoic acid [(S)-1-(2-hydroxy-4-methoxy-benzylcarbamoyl)-2-methyl-propyl]-amide (described in Example 2), and using the coupling conditions described in Example 1; ES-MS: 806.4, single peak at $t_R$= 8.36 min (System E).

Step 16.2: (2R,3R,4S)-4-((S)-2-Amino-3,3-dimethyl-butyrylamino)-2-(4-dimethylamino-benzylamino)-3-hydroxy-5-phenyl-pentanoic acid [(S)-1-(2-hydroxy-4-methoxy-benzylcarbamoyl)-2-methyl-propyl]-amide
The title compound is obtained starting from ((S)-1-{(1S,2R,3R)-1-benzyl-3-(4-dimethylamino-benzylamino)-2-hydroxy-3-[(S)-1-(2-hydroxy-4-methoxy-benzylcarbamoyl)-2-methylpropylcarbamoyl]-propylcarbamoyl}-2,2-dimethyl-propyl)-carbamic acid *tert*-butyl ester as described in Example 1; ES-MS: 706.4; single peak at $t_R$= 7.07 min (System E).

Analytical HPLC conditions:

| System | Gradient | Column | Flow rate | Temp. |
|---|---|---|---|---|
| (A) | 20-100% $CH_3CN$ in 13min + 5min 100% $CH_3CN$ (0.1 % TFA); detection at 215 nm | Nucleosil C-18 Dimension: 4.6 x 250 mm | 1.0 ml/min | 30 °C |
| (B) | 20-100% $CH_3CN$ in 13min + 5min 100% $CH_3CN$ (0.1 % TFA); detection at 215 nm | Nucleosil C-18 Dimension: 4.6 x 250 mm | 1.0 ml/min | 30 °C |
| (C) | 20-100% $CH_3CN$ in 7min + 2min 100% $CH_3CN$ (0.1% TFA); detection at 215 nm | Nucleosil 100-3 C-18 HD Dimension: 4.0 x 125 mm | 1.0 ml/min | 30 °C |
| (D) | 20-100% $CH_3CN$ in 7min + 3min 100% $CH_3CN$ (0.1 % TFA); detection at 215 nm | Nucleosil 100-3 C18 HD (125 x 4.6 mm) | 1.0 ml/min | 30 °C |
| (E) | linear gradient over 7 min of MeCN/0.09% TFA and $H_2O$/0.1% TFA from 1:49 to 1:0 and 3 min at 1:0; detection at 215 nm | Nucleosil $C_{18}$-column (250 x 4.6 mm, 5 μm, 100 A) | 2.0 ml/min | Room temp. |
| (F) | linear gradient over 7 min of MeCN/0.09% TFA and $H_2O$/0.1% TFA from 1:49 to 1:0 and 3 min at 1:0; detection at 215 nm | Nucleosil $C_{18AB}$-column (125 x 3 mm, 3 μm, 120 A) | 1.15 ml/min | Room temp. |
| (G) | linear gradient over 10 min of MeCN/0.09% TFA and $H_2O$/0.1% TFA from 1:49 to 1:0; detection at 215 nm | Nucleosil $C_{18}$-column (250 x 4 mm, 5 μm, 100 Å) | 2.0 ml/min | Room temp. |

Table continued

| System | Gradient | Column | Flow rate | Temp. |
|---|---|---|---|---|
| (H) | linear gradient over 10 min of MeCN/0.09% TFA and $H_2O$/0.1% TFA from 1:49 to 1:0; detection at 215 nm | Nucleosil $C_{18AB}$-column (125 x 3 mm, 3 μm, 120 A) | 1.15 ml/min | Room temp. |

Example 17: Inhibition of the chymotrypsin-like activity of the 20S proteasome

Exemplary $IC_{50}$ values determined according to the test described above for the compounds of formula I are given below (Table 1).

Table 1

| Example | $IC_{50}$ [μM] | | Example | $IC_{50}$ [μM] |
|---|---|---|---|---|
| 1 | 0.26 | | 9 | 0.19 |
| 2 | 0.6 | | 10 | 0.2 |
| 3 | 0.8 | | 11 | 1.04 |
| 4 | 1.5 | | 12 | 0.011 |
| 5 | 1.6 | | 13 | 0.018 |
| 6 | 0.05 | | 14 | 0.029 |
| 7 | 0.1 | | 15 | 0.15 |
| 8 | 0.15 | | 16 | 0.16 |

Example 18: Composition for oral Application

20.0 g of a solution for oral application can be prepared as follows (% means weight ingredient / total weight solution):

| | |
|---|---|
| Cremophor RH 40® | 9.6 g (48 %), |
| cornoil-mono-di-tri-glycerides | 5.8 g (29 %), |
| propylene glycol | 3.8 g (19 %), |
| compound of formula I | 0.8 g (4 %). |

The solution is kept at room temperature until use.

## Claims

**1.** A compound of formula I

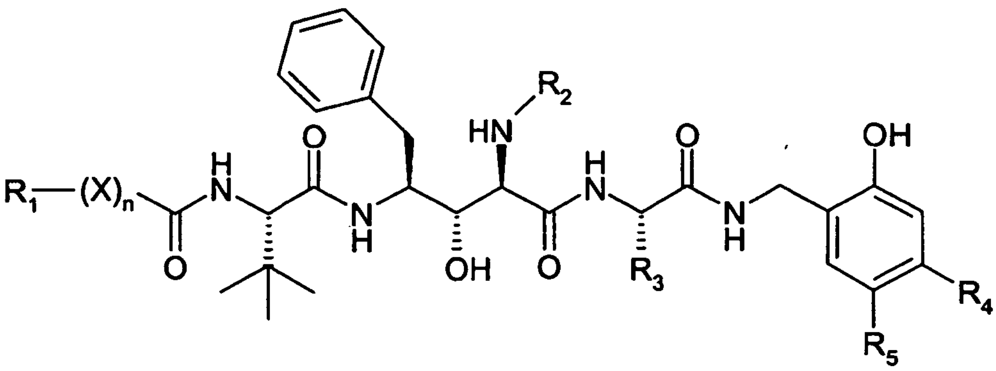

(I),

wherein
n is 0 or 1;
$R_1$ and $R_2$ are independently of the other an aliphatic radical, or an aromatic, aromatic-aliphatic, cycloaliphatic, cycloaliphatic-aliphatic, heterocyclic or heterocyclic-aliphatic radical, each radical having not more than 20 carbon atoms;
$R_3$ is hydrogen, oxa-alkyl, an aliphatic radical or a radical with up to 20 carbon atoms of the formula $-(Y)_m-R_6$, wherein Y is alkyl, m is 0 or 1 and $R_6$ is an unsubstituted or substituted monocyclic radical with 5 or 6 ring members containing up to 3 hetero atoms selected from the group consisting of nitrogen, oxygen and sulfur, wherein said monocyclic radical can also be fused to a benzo ring;
$R_4$ and $R_5$ are independently selected from the group consisting of hydrogen; an aliphatic radical; free, etherified or esterified hydroxy; free or esterified carboxy; formyl; alkanoyl; unsubstituted, mono- or di-substituted amino; mercapto; sulfo; alkyl-thio; carbamoyl; *N*-alkyl-carbamoyl; *N,N*-di-alkyl-carbamoyl; cyano and nitro; wherein carbon containing radicals $R_4$ and $R_5$ have up to 12 carbon atoms
X is nitrogen, oxygen or sulfur; and
if one of the radicals $R_4$ or $R_5$ is hydrogen, the other is as defined above for $R_4$ and $R_5$ with the exception of hydrogen, and if $R_4$ is methoxy, $R_2$ is as defined above with the exception of benzyl monosubstituted at position 4 by halogen, alkyl, alkoxy or hydroxyalkoxy; or a salt thereof.

**2.** A compound of formula I according to claim 1, wherein
n is 0 or 1;
$R_1$ has not more than 20 carbon atoms and is a member selected from the group consisting of $C_1$-$C_7$ alkyl, cycloalkyl-$C_1$-$C_7$ alkyl, phenyl- $C_1$-$C_7$ alkyl, naphthyl- $C_1$-$C_7$ alkyl, and chinolyl-$C_1$-$C_7$ alkyl, the members being unsubstituted or substituted by one or more radicals independently selected from the group consisting of hydroxy, $C_1$-$C_7$ alkoxy, $C_1$-$C_7$ alkanoyloxy, halogen, carboxy, $C_1$-$C_7$ alkoxycarbonyl, formyl, $C_1$-$C_7$ alkanoyl, amino, *N*-$C_1$-$C_7$ alkylamino, *N,N*-di-$C_1$-$C_7$ alkylamino, mercapto, sulfo, $C_1$-$C_7$ alkyl-thio, carbamoyl, *N*-$C_1$-$C_7$ alkyl-carbamoyl, *N,N*-di-$C_1$-$C_7$ alkyl-carbamoyl, cyano, nitro and $C_1$-$C_7$ alkyl that can itself be substituted by said radicals;
$R_2$ has not more than 20 carbon atoms and is a member selected from the group consisting of phenyl-$C_1$-$C_7$ alkyl, naphthyl-$C_1$-$C_7$ alkyl, chinolyl-$C_1$-$C_7$ alkyl, indolyl-$C_1$-$C_7$ alkyl and *N*-$C_{1}$-$C_7$ alkyl-indolyl-$C_1$-$C_7$ alkyl, the members being unsubstituted or substituted by one or more radicals independently selected from the group consisting of hydroxy, $C_1$-$C_7$ alkoxy, $C_1$-$C_7$ alkanoyloxy, halogen, carboxy, $C_1$-$C_7$ alkoxycarbonyl, formyl, $C_1$-$C_7$ alkanoyl, amino, *N*-$C_1$-$C_7$ alkylamino, *N,N*-di-$C_1$-$C_7$ alkylamino, mercapto, sulfo, $C_1$-$C_7$ alkyl-thio, carbamoyl, *N*-$C_1$-$C_7$ alkyl-carbamoyl, *N,N*-di-$C_1$-$C_7$ alkyl-carbamoyl, cyano, nitro and $C_1$-$C_7$ alkyl that can itself be substituted by said radicals;
$R_3$ is hydrogen, a radical of the formula $-G(-O-CH_2-CH_2)_t-R_7$, in which G and $R_7$ are $C_1$-$C_7$ alkyl and t is 1 to 3, unsubstituted $C_1$-$C_7$ alkyl or $C_1$-$C_7$ alkyl substituted by hydroxy, carboxy, amino, carbamoyl, guanidino, mercapto or $C_1$-$C_7$ alkyl-thio and having not more than 12 carbon atoms, or a radical of the formula $-(Y)_m-R_6$, wherein Y is $C_1$-$C_7$ alkyl, m is 0 or 1 and $R_6$ is phenyl, hydroxy-phenyl, imidazolyl, indolyl, piperidyl, piperazinyl, morpholinyl or pyridyl; $R_4$ and $R_5$ are independently selected from the group consisting of hydrogen, hydroxy, $C_1$-$C_7$ alkoxy, $C_1$-$C_7$ alkanoyloxy, halogen, carboxy, $C_1$-$C_7$ alkoxycarbonyl, formyl, $C_1$-$C_7$ alkanoyl, amino, *N*-$C_1$-$C_7$ alkylamino, *N,N*-di-$C_1$-$C_7$ alkylamino, mercapto, sulfo, $C_1$-$C_7$ alkyl-thio, carbamoyl, *N*-$C_1$-$C_7$ alkyl-carbamoyl, *N,N*-di-$C_1$-$C_7$ alkyl-carbamoyl, cyano, nitro and $C_1$-$C_7$ alkyl that can itself be substituted by said radicals, wherein carbon containing radicals $R_4$ and $R_5$ have up to 12 carbon atoms if ; and
X is nitrogen, oxygen or sulfur; and

if one of the radicals $R_4$ or $R_5$ is hydrogen, the other is as defined above for $R_4$ and $R_5$ with the exception of hydrogen, and if $R_4$ is methoxy, $R_2$ is as defined above with the exception of benzyl monosubstituted at position 4 by halogen, alkyl, alkoxy or hydroxyalkoxy;
or a salt thereof.

**3.** A compound of formula I according to claim 1, wherein
n is 0 or 1;
$R_1$ has not more than 20 carbon atoms and is a member selected from the group consisting of $C_1$-$C_7$ alkyl, cycloalkyl-$C_1$-$C_7$ alkyl, phenyl-$C_1$-$C_7$ alkyl, naphthyl-$C_1$-$C_7$ alkyl, and chinolyl-$C_1$-$C_7$ alkyl, the members being unsubstituted or substituted by one or more radicals independently selected from the group consisting of an aliphatic radical; free, etherified or esterified hydroxy; free or esterified carboxy; formyl; alkanoyl; unsubstituted, mono- or di-substituted amino; mercapto; sulfo; alkyl-thio; carbamoyl; *N*-alkyl-carbamoyl; *N,N*-di-alkyl-carbamoyl; cyano and nitro;
$R_2$ has not more than 20 carbon atoms and is a member selected from the group consisting of phenyl-$C_1$-$C_7$ alkyl, naphthyl-$C_1$-$C_7$ alkyl, chinolyl-$C_1$-$C_7$ alkyl, indolyl-$C_1$-$C_7$ alkyl and *N*-$C_1$-$C_7$ alkyl-indolyl-$C_1$-$C_7$ alkyl, the members being unsubstituted or substituted by one or more radicals independently selected from the group consisting of an aliphatic radical; free, etherified or esterified hydroxy; free or esterified carboxy; formyl; alkanoyl; unsubstituted, mono- or di-substituted amino; mercapto; sulfo; alkyl-thio; carbamoyl; *N*-alkyl-carbamoyl; N,N-di-alkyl-carbamoyl; cyano and nitro;
$R_3$ is hydrogen, oxa-alkyl, an aliphatic radical or a radical with up to 20 carbon atoms of the formula $-(Y)_m-R_6$, wherein Y is alkyl, m is 0 or 1 and $R_6$ is an unsubstituted or substituted monocyclic radical with 5 or 6 ring members containing up to 3 hetero atoms selected from the group consisting of nitrogen, oxygen and sulfur, wherein said monocyclic radical can also be fused to a benzo ring;
$R_4$ and $R_5$ are independently selected from the group consisting of hydrogen; an aliphatic radical; free, etherified or esterified hydroxy; free or esterified carboxy; formyl; alkanoyl; unsubstituted, mono- or di-substituted amino; mercapto; sulfo; alkyl-thio; carbamoyl; N-alkyl-carbamoyl; *N,N*-di-alkyl-carbamoyl; cyano and nitro; wherein carbon containing radicals $R_4$ and $R_5$ have up to 12 carbon atoms ; and
X is oxygen; and
if one of the radicals $R_4$ or $R_5$ is hydrogen, the other is as defined above for $R_4$ and $R_5$ with the exception of hydrogen, and if $R_4$ is methoxy, $R_2$ is as defined above with the exception of benzyl monosubstituted at position 4 by halogen, alkyl, alkoxy or hydroxyalkoxy;
or a salt thereof.

**4.** A compound of formula I according to claim 1, wherein
n is 0 or 1;
R, and $R_2$ are independently of the other an aliphatic radical, or an aromatic, aromatic-aliphatic, cycloaliphatic, cycloaliphatic-aliphatic, heterocyclic or heterocyclic-aliphatic radical, each radical having not more than 20 carbon atoms;
$R_3$ is hydrogen, a radical of the formula $-G(-O-CH_2-CH_2)_t-R_7$, in which G and $R_7$ are $C_1$-$C_7$ alkyl and t is 1 to 3, unsubstituted $C_1$-$C_7$ alkyl or $C_1$-$C_7$ alkyl substituted by hydroxy, carboxy, amino, carbamoyl, guanidino, mercapto or $C_1$-$C_7$ alkyl-thio and having not more than 12 carbon atoms, or a radical of the formula $-(Y)_m-R_6$, wherein Y is $C_1$-$C_7$ alkyl, m is 0 or 1 and $R_6$ is phenyl, hydroxy-phenyl, imidazolyl, indolyl, piperidyl, piperazinyl, morpholinyl or pyridyl; $R_4$ and $R_5$ are independently selected from the group consisting of hydrogen; an aliphatic radical; free, etherified or esterified hydroxy; free or esterified carboxy; formyl; alkanoyl; unsubstituted, mono- or di-substituted amino; mercapto; sulfo; alkyl-thio; carbamoyl; N-alkyl-carbamoyl; *N,N*-di-alkyl-carbamoyl; cyano and nitro; wherein carbon containing radicals $R_4$ and $R_5$ have up to 12 carbon atoms and
X is oxygen; and
if one of the radicals $R_4$ or $R_5$ is hydrogen, the other is as defined above for $R_4$ and $R_5$ with the exception of hydrogen, and if $R_4$ is methoxy, $R_2$ is as defined above with the exception of benzyl monosubstituted at position 4 by halogen, alkyl, alkoxy or hydroxyalkoxy; or a salt thereof.

**5.** A compound of formula I according to claim 1, wherein
n is 0;
$R_1$ and $R_2$ are independently of the other an aliphatic radical, or an aromatic, aromatic-aliphatic, cycloaliphatic, cycloaliphatic-aliphatic, heterocyclic or heterocyclic-aliphatic radical, each radical having not more than 20 carbon atoms;
$R_3$ is hydrogen, oxa-alkyl, an aliphatic radical or a radical with up to 20 carbon atoms of the formula $-(Y)_m-R_6$, wherein Y is alkyl, m is 0 or 1 and $R_6$ is an unsubstituted or substituted monocyclic radical with 5 or 6 ring members containing up to 3 hetero atoms selected from the group consisting of nitrogen, oxygen and sulfur, wherein said monocyclic

radical can also be fused to a benzo ring; and
$R_4$ and $R_5$ are independently selected from the group consisting of hydrogen; an aliphatic radical; free, etherified or esterified hydroxy; free or esterified carboxy; formyl; alkanoyl; unsubstituted, mono- or di-substituted amino; mercapto; sulfo; alkyl-thio; carbamoyl; N-alkyl-carbamoyl; *N,N*-di-alkyl-carbamoyl; cyano and nitro; wherein carbon containing radicals $R_4$ and $R_5$ have up to 12 carbon atoms is and
if one of the radicals $R_4$ or $R_5$ is hydrogen, the other is as defined above for $R_4$ and $R_5$ with the exception of hydrogen, and if $R_4$ is methoxy, $R_2$ is as defined above with the exception of benzyl monosubstituted at position 4 by halogen, alkyl, alkoxy or hydroxyalkoxy;
or a salt thereof.

**6.** A compound of formula I according to claim 1, wherein
n is 0 or 1;
$R_1$ is $C_1$-$C_7$ alkyl, phenyl- $C_1$-$C_7$ alkyl or naphthyl-$C_1$-$C_7$ alkyl;
$R_2$ is mono-, di-, or tri-$C_1$-$C_7$ alkoxy-phenyl-$C_1$-$C_7$ alkyl, *N,N*-di-$C_1$-$C_7$ alkyl-amino-phenyl-$C_{1-}C_7$ alkyl or naphthyl-$C_1$-$C_7$ alkyl;
$R_3$ is $C_1$-$C_7$ alkyl;
$R_4$ and $R_5$ are independently selected from the group consisting of hydrogen, $C_1$-$C_7$ alkoxy and halogen is ; and
X is oxygen; and
if one of the radicals $R_4$ or $R_5$ is hydrogen, the other is as defined above for $R_4$ and $R_5$ with the exception of hydrogen, and if $R_4$ is methoxy, $R_2$ is as defined above with the exception of benzyl monosubstituted at position 4 by halogen, alkyl, alkoxy or hydroxyalkoxy;
or a salt thereof.

**7.** A compound of formula I according to claim 1 selected from the group consisting of
((S)-1-{(1S,2R,3R)-1-benzyl-2-hydroxy-3-[(S)-1-(2-hydroxy-4-methoxy-benzylcarbamoyl)-2-methyl-propylcarbamoyl]-3-[(naphthalen-1-ylmethyl)-amino]-propylcarbamoyl}-2,2-dimethylpropyl)-carbamic acid benzyl ester,
((S)-1-{(1S,2R,3R)-1-benzyl-3-(4-dimethylamino-benzylamino)-2-hydroxy-3-[(S)-1-(2-hydroxy-4-methoxy-benzylcarbamoyl)-2-methyl-propylcarbamoyl]-propylcarbamoyl}-2,2-dimethyl-propyl)-carbamic acid benzyl ester,
{(S)-1-[(1S,2R,3R)-1-benzyl-3-[(S)-1-(5-bromo-2-hydroxy-benzyl-carbamoyl)-2-methylpropylcarbamoyl]-2-hydroxy-3-(4-methoxy-benzylamino)-propylcarbamoyl]-2,2-dimethylpropyl}-carbamic acid benzyl ester,
{(S)-1-[(1S,2R,3R)-1-benzyl-2-hydroxy-3-[(S)-1-(2-hydroxy-4-methoxy-benzylcarbamoyl)-2-methyl-propylcarbamoyl]-3-(3-methoxy-benzylamino)-propylcarbamoyl]-2,2-dimethyl-propyl}-carbamic acid benzyl ester,
((S)-1-{(1S,2R,3R)-1-benzyl-3-(2,4-dimethoxy-benzylamino)-2-hydroxy-3-[(S)-1-(2-hydroxy-4-methoxy-benzylcarbamoyl)-2-methyl-propylcarbamoyl]-propylcarbamoyl}-2,2-dimethylpropyl)-carbamic acid benzyl ester,
{(S)-1-[(1S,2R,3R)-1-benzyl-2-hydroxy-3-[(S)-1-(2-hydroxy-4-methoxy-benzylcarbamoyl)-2-methyl-propylcarbamoyl]-3-(3,4,5-trimethoxy-benzylamino)-propylcarbamoyl]-2,2-dimethylpropyl}-carbamic acid benzyl ester,
{(S)-1-[(1S,2R,3R)-1-benzyl-2-hydroxy-3-[(S)-1-(2-hydroxy-4-methoxy-benzylcarbamoyl)-2-methyl-propylcarbamoyl]-3-(2,3,4-trimethoxy-benzylamino)-propylcarbamoyl]-2,2-dimethylpropyl}-carbamic acid benzyl ester,
((S)-1-{(1S,2R,3R)-1-benzyl-3-(3,4-dimethoxy-benzylamino)-2-hydroxy-3-[(S)-1-(2-hydroxy-4-methoxy-benzylcarbamoyl)-2-methyl-propylcarbamoyl]-propylcarbamoyl}-2,2-dimethylpropyl)-carbamic acid benzyl ester,
((S)-1-{(1S,2R,3R)-1-benzyl-3-(3,5-dimethoxy-benzyiamino)-2-hydroxy-3-[(S)-1-(2-hydroxy-4-methoxy-benzylcarbamoyl)-2-methyl-propylcarbamoyl]-propylcarbamoyl}-2,2-dimethylpropyl)-carbamic acid benzyl ester,
{(S)-1-[(1S,2R,3R)-1-benzyl-2-hydroxy-3-[(S)-1-(2-hydroxy-4-methoxy-benzylcarbamoyl)-2-methyl-propylcarbamoyl]-3-(2,4,5-trimethoxy-benzylamino)-propylcarbamoyl]-2,2-dimethylpropyl}-carbamic acid benzyl ester,
{(S)-1-[(1S,2R,3R)-1-benzyl-2-hydroxy-3-[(S)-1-(2-hydroxy-4-methoxy-benzylcarbamoyl)-2-methyl-propylcarbamoyl]-3-(3,4,5-trimethoxy-benzylamino)-propylcarbamoyl]-2,2-dimethylpropyl}-carbamic acid *tert*-butyl ester,
(2R,3R,4S)-4-[(S)-3,3-dimethyl-2-(2-naphthalen-1-yl-acetylamino)-butyrylamino]-3-hydroxy-5-phenyl-2-(3,4,5-trimethoxy-benzylamino)-pentanoic acid [(S)-1-(2-hydroxy-4-methoxy-benzylcarbamoyl)-2-methyl-propyl]-amide,
(2R,3R,4S)-4-[(S)-3,3-dimethyl-2-(2-naphthalen-1-yl-acetylamino)-butyrylamino]-3-hydroxy-5-phenyl-2-(2,3,4-trimethoxy-benzylamino)-pentanoic acid [(S)-1-(2-hydroxy-4-methoxy-benzylcarbamoyl)-2-methyl-propyl]-amide,
(2R,3R,4S)-4-[(S)-3,3-dimethyl-2-(2-naphthalen-1-yl-acetylamino)-butyrylamino]-3-hydroxy-5-phenyl-2-(2,4,5-trimethoxy-benzylamino)-pentanoic acid [(S)-1-(2-hydroxy-4-methoxy-benzylcarbamoyl)-2-methyl-propyl]-amide,
(2R,3R,4S)-4-[(S)-3,3-dimethyl-2-(2-naphthalen-1-yl-acetylamino)-butyrylamino]-3-hydroxy-2-[(naphthalen-1-ylmethyl)-amino]-5-phenyl-pentanoic acid [(S)-1-(2-hydroxy-4-methoxy-benzylcarbamoyl)-2-methyl-propyl]-amide,
(2R,3R,4S)-2-(4-dimethylamino-benzylamino)-4-[(S)-3,3-dimethyt-2-(2-naphthalen-1-yl-acetylamino)-butyrylamino]-3-hydroxy-5-phenyl-pentanoic acid [(S)-1-(2-hydroxy-4-methoxy-benzylcarbamoyl)-2-methyl-propyl]-amide,
and pharmaceutically acceptable salts thereof.

8. A pharmaceutically acceptable salt of a compound of formula I according to any one of claims 1-6.

9. A compound of formula I according to any one of claims 1 to 7 or a pharmaceutically acceptable salt of such a compound for use in a method for the therapeutic treatment of the human or animal body.

10. A pharmaceutical composition comprising a therapeutically effective amount of a compound of formula I according to any one of claims 1 to 7 or a pharmaceutically acceptable salt of such a compound together with a pharmaceutical carrier.

11. A pharmaceutical composition for the treatment of proliferative diseases in warm-blooded animals, including humans, comprising, in a dose effective against the disease, a compound of formula I according to any one of claims 1 to 7, or a pharmaceutically acceptable salt of such a compound, together with a pharmaceutical carrier.

12. The use of a compound of formula I according to any one of claims 1 to 7 or a pharmaceutically acceptable salt of such a compound for the preparation of pharmaceutical compositions for use in the treatment of proliferative diseases.

13. A process for the preparation of a compound of formula I according to claim 1 or of a salt of such a compound, **characterized in that**

a) a compound of formula II

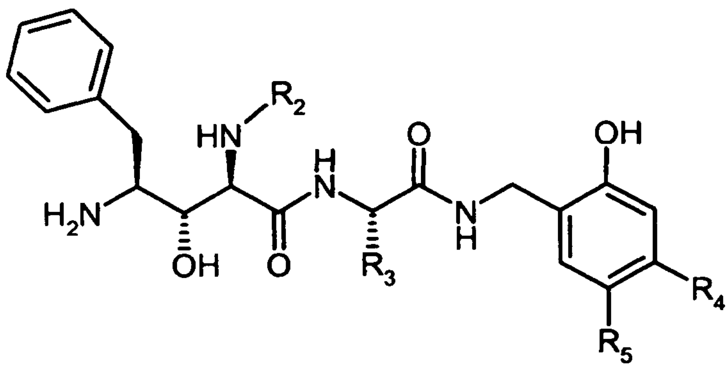

(II),

wherein $R_2$, $R_3$, $R_4$ and $R_5$ are as defined for compounds of formula I according to claim 1, functional groups present in a compound of formula II, with the exception of the groups participating in the reaction, being protected if necessary by readily removable protecting groups, or a salt of such a compound, is reacted with an acid of formula III

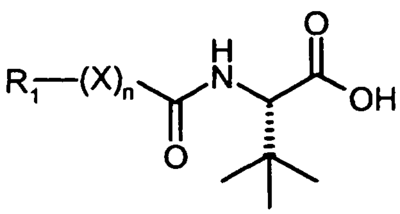

(III),

wherein n, $R_1$ and X are as defined for compounds of formula I according to claim 1, functional groups present in a compound of formula III, with the exception of the groups participating in the reaction, being protected if necessary by readily removable protecting groups, or a suitable reactive acid derivative of such a compound, and any protecting groups present are removed, or

b) a compound of formula IV

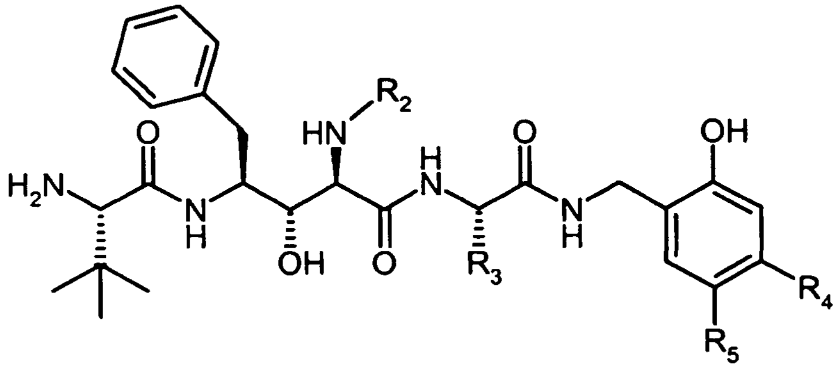

(IV),

wherein $R_2$, $R_3$, $R_4$ and $R_5$ are as defined for compounds of formula I according to claim 1, functional groups present in a compound of formula IV, with the exception of the groups participating in the reaction, being protected if necessary by readily removable protecting groups, or a salt of such a compound, is reacted with an acid of formula V

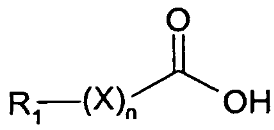

(V),

wherein n, $R_1$ and X are as defined for compounds of formula I according to claim 1, functional groups present in a compound of formula V, with the exception of the groups participating in the reaction, being protected if necessary by readily removable protecting groups, or a suitable reactive acid derivative of such a compound, and any protecting groups present are removed,
and, if desired, a compound of formula I obtained by process a) or b) is converted into another compound of formula I, an obtained free compound of formula I is converted into a salt, or an obtained salt of a compound of formula I is converted into another salt or into its free form.

**Patentansprüche**

1. Verbindung der Formel I

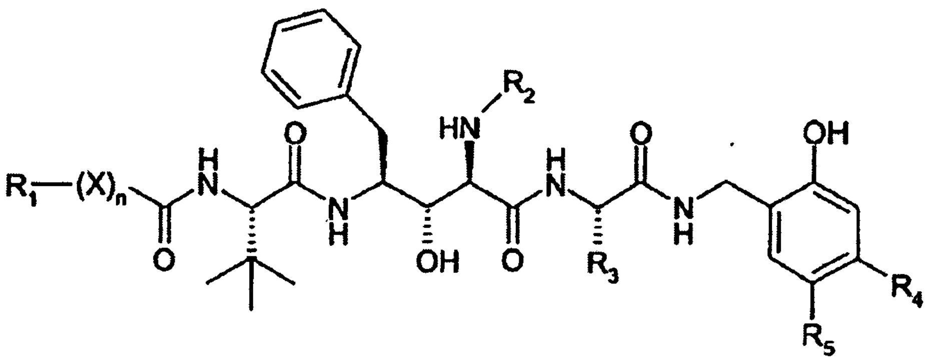

(I),

worin n für 0 oder 1 steht,
$R_1$ und $R_2$ unabhängig voneinander für einen aliphatischen Rest oder einen aromatischen, aromatisch-aliphatischen, cycloaliphatischen, cycloaliphatisch-aliphatischen, heterocyclischen oder heterocyclischaliphatischen Rest stehen, wobei jeder Rest nicht mehr als 20 Kohlenstoffatome aufweist,

$R_3$ für Wasserstoff, Oxaalkyl, einen aliphatischen Rest oder einen Rest mit bis zu 20 Kohlenstoffatomen der Formel $-(Y)_m-R_6$ steht, worin Y für Alkyl steht, m für 0 oder 1 steht und $R_6$ für einen unsubstituierten oder substituierten monocyclischen Rest mit 5 oder 6 Ringatomen steht, die bis zu 3 Heteroatome enthalten, welche aus der Gruppe ausgewählt sind, die besteht aus Stickstoff, Sauerstoff und Schwefel, worin der monocyclische Rest auch an einen Benzoring fusioniert sein kann,
$R_4$ und $R_5$ unabhängig aus der Gruppe ausgewählt sind, die besteht aus Wasserstoff, einem aliphatischen Rest, freiem, verethertem oder verestertem Hydroxy, freiem oder verestertem Carboxy, Formyl, Alkanoyl, unsubstituiertem, mono- oder disubstituiertem Amino, Mercapto, Sulfo, Alkylthio, Carbamoyl, N-Alkylcarbamoyl, N,N-Dialkylcarbamoyl, Cyano und Nitro, worin die Kohlenstoff-enthaltenden Reste $R_4$ und $R_5$ bis zu 12 Kohlenstoffatome aufweisen,
X für Stickstoff, Sauerstoff oder Schwefel steht, und falls einer der Reste $R_4$ und $R_5$ für Wasserstoff steht, der andere wie oben für $R_4$ und $R_5$ definiert ist, mit der Ausnahme von Wasserstoff und falls $R_4$ für Methoxy steht, $R_2$ wie oben definiert ist mit Ausnahme von Benzyl, das an der Position 4 durch Halogen, Alkyl, Alkoxy oder Hydroxyalkoxy monosubstituiert ist,
oder ein Salz hiervon.

**2.** Verbindung der Formel I nach Anspruch 1, worin
n für 0 oder 1 steht,
$R_1$ nicht mehr als 20 Kohlenstoffatome aufweist und ein Vertreter ist, der aus der Gruppe ausgewählt ist, welche besteht aus $C_1$-$C_7$ Alkyl, Cycloalkyl-$C_1$-$C_7$-alkyl, Phenyl-$C_1$-$C_7$-alkyl, Naphthyl-$C_1$-$C_7$-alkyl und Chinolyl-$C_1$-$C_7$-alkyl, wobei die Vertreter unsubstituiert oder durch einen oder mehrere Reste substituiert sind, die unabhängig aus der Gruppe ausgewählt sind, die besteht aus Hydroxy, $C_1$-$C_7$ Alkoxy, $C_1$-$C_7$ Alkanoyloxy, Halogen, Carboxy, $C_1$-$C_7$ Alkoxycarbonyl, Formyl, $C_1$-$C_7$ Alkanoyl, Amino, N-$C_1$-$C_7$ Alkylamino, N,N-Di-$C_1$-$C_7$ Alkylamino, Mercapto, Sulfo, $C_1$-$C_7$ Alkylthio, Carbamoyl, N-$C_1$-$C_7$ Alkylcarbamoyl, N,N-Di-$C_1$-$C_7$ Alkylcarbamoyl, Cyano, Nitro und $C_1$-$C_7$ Alkyl, das selbst durch diese Reste substituiert sein kann,
$R_2$ nicht mehr als 20 Kohlenstoffatome aufweist und ein Vertreter ist, der aus der Gruppe ausgewählt ist, welche besteht aus Phenyl-$C_1$-$C_7$-alkyl, Naphthyl-$C_1$-$C_7$-alkyl, Chinolyl-$C_1$-$C_7$-alkyl, Indolyl-$C_1$-$C_7$-alkyl und N-$C_1$-$C_7$ Alkylindolyl-$C_1$-$C_7$-alkyl, wobei die Vertreter unsubstituiert oder durch einen oder mehrere Reste substituiert sind, die unabhängig aus der Gruppe ausgewählt sind, die besteht aus Hydroxy, $C_1$-$C_7$ Alkoxy, $C_1$-$C_7$ Alkanoyloxy, Halogen, Carboxy, $C_1$-$C_7$ Alkoxycarbonyl, Formyl, $C_1$-$C_7$ Alkanoyl, Amino, N-$C_1$-$C_7$ Alkylamino, N,N-Di-$C_1$-$C_7$ Alkylamino, Mercapto, Sulfo, $C_1$-$C_7$ Alkylthio, Carbamoyl, N-$C_1$-$C_7$ Alkylcarbamoyl, N,N-Di-$C_1$-$C_7$ Alkylcarbamoyl, Cyano, Nitro und $C_1$-$C_7$ Alkyl, das selbst durch diese Reste substituiert sein kann,
$R_3$ steht für Wasserstoff, einen Rest der Formel $-G(-O-CH_2-CH_2)_t-R_7$, worin G und $R_7$ für $C_1$-$C_7$ Alkyl stehen und t für 1 bis 3 steht, für unsubstituiertes $C_1$-$C_7$ Alkyl oder $C_1$-$C_7$ Alkyl, das substituiert ist mit Hydroxy, Carboxy, Amino, Carbamoyl, Guanidino, Mercapto oder $C_1$-$C_7$ Alkylthio und nicht mehr als 12 Kohlenstoffatome aufweist oder für einen Rest der Formel $-(Y)_m-R_6$, worin Y für $C_1$-$C_7$ Alkyl steht, m für 0 oder 1 steht und $R_6$ für Phenyl, Hydroxyphenyl, Imidazolyl, Indolyl, Piperidyl, Piperazinyl, Morpholinyl oder Pyridyl steht,
$R_4$ und $R_5$ unabhängig aus der Gruppe ausgewählt sind, die besteht aus Wasserstoff, Hydroxy, $C_1$-$C_7$ Alkoxy, $C_1$-$C_7$ Alkanoyloxy, Halogen, Carboxy, $C_1$-$C_7$ Alkoxycarbonyl, Formyl, $C_1$-$C_7$ Alkanoyl, Amino, N-$C_1$-$C_7$ Alkylamino, N,N-Di-$C_1$-$C_7$ Alkylamino, Mercapto, Sulfo, $C_1$-$C_7$ Alkylthio, Carbamoyl, N-$C_1$-$C_7$ Alkylcarbamoyl, N,N-Di-$C_1$-$C_7$ Alkylcarbamoyl, Cyano, Nitro und $C_1$-$C_7$ Alkyl, das selbst durch diese Reste substituiert sein kann, worin die Kohlenstoff enthaltenden Reste $R_4$ und $R_5$ bis zu 12 Kohlenstoffatome aufweisen,
X für Stickstoff, Sauerstoff oder Schwefel steht, und
falls einer der Reste $R_4$ oder $R_5$ für Wasserstoff steht, der andere wie oben für $R_4$ und $R_5$ mit Ausnahme von Wasserstoff definiert ist und falls $R_4$ für Methoxy steht, $R_2$ wie oben definiert ist mit der Ausnahme von Benzyl, das an der Position 4 durch Halogen, Alkyl, Alkoxy oder Hydroxyalkoxy monosubstituiert ist,
oder ein Salz hiervon.

**3.** Verbindung der Formel I nach Anspruch 1, worin
n für 0 oder 1 steht,
$R_1$ nicht mehr als 20 Kohlenstoffatome aufweist und ein Vertreter ist, der aus der Gruppe ausgewählt ist, welche besteht aus $C_1$-$C_7$ Alkyl, Cycloalkyl-$C_1$-$C_7$-alkyl, Phenyl-$C_1$-$C_7$-alkyl, Naphthyl-$C_1$-$C_7$-alkyl und Chinolyl-$C_1$-$C_7$-alkyl, wobei die Vertreter unsubstituiert oder durch einen oder mehrere Reste substituiert sind, die unabhängig aus der Gruppe ausgewählt sind, die besteht aus einem aliphatischen Rest, freiem, verethertem oder verestertem Hydroxy, freiem oder verestertem Carboxy, Formyl, Alkanoyl, unsubstituiertem, mono- oder disubstituiertem Amino, Mercapto, Sulfo, Alkylthio, Carbamoyl, N-Alkyl-carbamoyl, N,N-Dialkylcarbamoyl, Cyano und Nitro,
$R_2$ nicht mehr als 20 Kohlenstoffatome aufweist und ein Vertreter ist, der aus der Gruppe ausgewählt ist, welche besteht aus Phenyl-$C_1$-$C_7$-alkyl, Naphthyl-$C_1$-$C_7$-alkyl, Chinolyl-$C_1$-$C_7$-alkyl, Indolyl-$C_1$-$C_7$-alkyl und N-$C_1$-$C_7$ Alky-

lindolyl-$C_1$-$C_7$-alkyl, wobei die Vertreter unsubstituiert oder durch einen oder mehrere Reste substituiert sind, die unabhängig aus der Gruppe ausgewählt sind, die besteht aus einem aliphatischen Rest, freiem, verethertem oder verestertem Hydroxy, freiem oder verestertem Carboxy, Formyl, Alkanoyl, unsubstituiertem, mono- oder disubstituiertem Amino, Mercapto, Sulfo, Alkylthio, Carbamoyl, N-Alkylcarbamoyl, N,N-Dialkylcarbamoyl, Cyano und Nitro,
$R_3$ steht für Wasserstoff, Oxaalkyl, einen aliphatischen Rest oder einen Rest mit bis zu 20 Kohlenstoffatomen der Formel $-(Y)_m-R_6$, worin Y für Alkyl steht, m für 0 oder 1 steht und $R_6$ für einen unsubstituiterten oder substituierten monocyclischen Rest mit 5 oder 6 Ringgliedern steht, die bis zu 3 Heteroatome enthalten, welche aus der Gruppe ausgewählt sind, die besteht aus Stickstoff, Sauerstoff und Schwefel und worin der monocyclische Rest auch an einen Benzoring fusioniert sein kann,
$R_4$ und $R_5$ unabhängig aus der Gruppe ausgewählt sind, die besteht aus Wasserstoff, einem aliphatischen Rest, freiem, verethertem oder verestertem Hydroxy, freiem oder verestertem Carboxy, Formyl, Alkanoyl, unsubstituiertem, mono- oder disubstituiertem Amino, Mercapto, Sulfo, Alkylthio, Carbamoyl, N-Alkylcarbamoyl, N,N-Dialkylcarbamoyl, Cyano und Nitro, worin die Kohlenstoff-enthaltenden Reste $R_4$ und $R_5$ bis zu 12 Kohlenstoffatome aufweisen, und
X für Sauerstoff steht, und
falls einer der Reste $R_4$ oder $R_5$ für Wasserstoff steht, der andere wie oben für $R_4$ und $R_5$ mit Ausnahme von Wasserstoff definiert ist und falls $R_4$ für Methoxy steht, $R_2$ wie oben definiert ist mit der Ausnahme von Benzyl, das an der Position 4 durch Halogen, Alkyl, Alkoxy oder Hydroxyalkoxy monosubstituiert ist,
oder ein Salz hiervon.

**4.** Verbindung der Formel I nach Anspruch 1, worin
n für 0 oder 1 steht,
$R_1$ und $R_2$ unabhängig voneinander für einen aliphatischen Rest oder einen aromatischen, aromatischaliphatischen, cycloaliphatischen, cycloaliphatisch-aliphatischen, heterocyclischen oder heterocyclisch-aliphatischen Rest stehen, wobei jeder Rest nicht mehr als 20 Kohlenstoffatome aufweist,
$R_3$ steht für Wasserstoff, einen Rest der Formel $-G(-O-CH_2-CH_2)_t-R_7$, worin G und $R_7$ für $C_1$-$C_7$ Alkyl stehen und t für 1 bis 3 steht, für unsubstituiertes $C_1$-$C_7$ Alkyl oder $C_1$-$C_7$ Alkyl, das substituiert ist mit Hydroxy, Carboxy, Amino, Carbamoyl, Guanidino, Mercapto oder $C_1$-$C_7$ Alkylthio und nicht mehr als 12 Kohlenstoffatome aufweist oder für einen Rest der Formel $-(Y)_m-R_6$, worin Y für $C_1$-$C_7$ Alkyl steht, m für 0 oder 1 steht und $R_6$ für Phenyl, Hydroxyphenyl, Imidazolyl, Indolyl, Piperidyl, Piperazinyl, Morpholinyl oder Pyridyl steht,
$R_4$ und $R_5$ unabhängig aus der Gruppe ausgewählt sind, die besteht aus Wasserstoff, einem aliphatischen Rest, freiem, verethertem oder verestertem Hydroxy, freiem oder verestertem Carboxy, Formyl, Alkanoyl, unsubstituiertem, mono- oder disubstituiertem Amino, Mercapto, Sulfo, Alkylthio, Carbamoyl, N-Alkylcarbamoyl, N,N-Dialkylcarbamoyl, Cyano und Nitro, worin die Kohlenstoff-enthaltenden Reste $R_4$ und $R_5$ bis zu 12 Kohlenstoffatome aufweisen, und
X für Sauerstoff steht, und
falls einer der Reste $R_4$ oder $R_5$ für Wasserstoff steht, der andere wie oben für $R_4$ und $R_5$ mit Ausnahme von Wasserstoff definiert ist und falls $R_4$ für Methoxy steht, $R_2$ wie oben definiert ist mit der Ausnahme von Benzyl, das an der Position 4 durch Halogen, Alkyl, Alkoxy oder Hydroxyalkoxy monosubstituiert ist,
oder ein Salz hiervon.

**5.** Verbindung der Formel I nach Anspruch 1, worin
n für 0 steht,
$R_1$ und $R_2$ unabhängig voneinander für einen aliphatischen Rest oder einen aromatischen, aromatischaliphatischen, cycloaliphatischen, cycloaliphatisch-aliphatischen, heterocyclischen oder heterocyclisch-aliphatischen Rest stehen, wobei jeder Rest nicht mehr als 20 Kohlenstoffatome aufweist,
$R_3$ steht für Wasserstoff, Oxaalkyl, einen aliphatischen Rest oder einen Rest mit bis zu 20 Kohlenstoffatomen der Formel $-(Y)_m-R_6$, worin Y für Alkyl steht, m für 0 oder 1 steht und $R_6$ für einen unsubstituierten oder substituierten monocyclischen Rest mit 5 oder 6 Ringgliedem steht, die bis zu 3 Heteroatome enthalten, welche aus der Gruppe ausgewählt sind, die besteht aus Stickstoff, Sauerstoff und Schwefel und worin der monocyclische Rest auch an einen Benzoring fusioniert sein kann,
$R_4$ und $R_5$ unabhängig aus der Gruppe ausgewählt sind, die besteht aus Wasserstoff, einem aliphatischen Rest, freiem, verethertem oder verestertem Hydroxy, freiem oder verestertem Carboxy, Formyl, Alkanoyl, unsubstituiertem, mono- oder disubstituiertem Amino, Mercapto, Sulfo, Alkylthio, Carbamoyl, N-Alkylcarbamoyl, N,N-Dialkylcarbamoyl, Cyano und Nitro, worin die Kohlenstoff-enthaltenden Reste $R_4$ und $R_5$ bis zu 12 Kohlenstoffatome aufweisen, und
falls einer der Reste $R_4$ oder $R_5$ für Wasserstoff steht, der andere wie oben für $R_4$ und $R_5$ mit Ausnahme von Wasserstoff definiert ist und falls $R_4$ für Methoxy steht, $R_2$ wie oben definiert ist mit der Ausnahme von Benzyl, das

an der Position 4 durch Halogen, Alkyl, Alkoxy oder Hydroxyalkoxy monosubstituiert ist,
oder ein Salz hiervon.

**6.** Verbindung der Formel I nach Anspruch 1, worin
n für 0 oder 1 steht,
$R_1$ für $C_1$-$C_7$ Alkyl, Phenyl-$C_1$-$C_7$-alkyl oder Naphthyl-$C_1$-$C_7$-alkyl steht,
$R_2$ für Mono-, Di- oder Tri-$C_1$-$C_7$-alkoxyphenyl-$C_1$-$C_7$-alkyl, N,N-Di-$C_1$-$C_7$-alkylaminophenyl-$C_1$-$C_7$-alkyl oder Naphthyl-$C_1$-$C_7$-alkyl steht,
$R_3$ für $C_1$-$C_7$ Alkyl steht,
$R_4$ und $R_5$ unabhängig aus der Gruppe ausgewählt sind, die besteht aus Wasserstoff, $C_1$-$C_7$ Alkoxy und Halogen, und
X für Sauerstoff steht, und
falls einer der Reste $R_4$ oder $R_5$ für Wasserstoff steht, der andere wie oben für $R_4$ und $R_5$ mit Ausnahme von Wasserstoff definiert ist und falls $R_4$ für Methoxy steht, $R_2$ wie oben definiert ist mit der Ausnahme von Benzyl, das an der Position 4 durch Halogen, Alkyl, Alkoxy oder Hydroxyalkoxy monosubstituiert ist,
oder ein Salz hiervon.

**7.** Verbindung der Formel I nach Anspruch 1, die aus der Gruppe ausgewählt ist, welche besteht aus
((S)-1-{(1S,2R,3R)-1-Benzyl-2-hydroxy-3-[(S)-1-(2-hydroxy-4-methoxybenzylcarbamoyl)-2-methylpropyl-carbamoyl]-3-[(naphthalin-1-ylmethyl)-amino]propylcarbamoyl}-2,2-dimethylpropyl)carbaminsäure-benzylester,
((S)-1-{(1S,2R,3R)-1-Benzyl-3-(4-dimethylaminobenzylamino)-2-hydroxy-3-[(S)-1-(2-hydroxy-4-methoxy-benzylcarbamoyl)-2-methylpropylcarbamoyl]propylcarbamoyl}-2,2-dimethylpropyl)carbaminsäure-benzylester,
{(S)-1-[(1S,2R,3R)-1-Benzyl-3-[(S)-1-(5-brom-2-hydroxybenzylcarbamoyl)-2-methylpropylcarbamoyl]-2-hydroxy-3-(4-methoxybenzylamino)propylcarbamoyl]-2,2-dimethylpropyl}carbaminsäurebenzylester,
{(S)-1-[(1S,2R,3R)-1-Benzyl-2-hydroxy-3-[(S)-1-(2-hydroxy-4-methoxybenzylcarbamoyl)-2-methylpropyl-carbamoyl]-3-(3-methoxybenzylamino)propylcarbamoyl]-2,2-dimethylpropyl}carbaminsäurebenzyl-ester,
((S)-1-{(1S,2R,3R)-1-Benzyl-3-(2,4-dimethoxybenzylamino)-2-hydroxy-3-[(S)-1-(2-hydroxy-4-methoxy-benzylcarbamoyl)-2-methylpropylcarbamoyl]propylcarbamoyl}-2,2-dimethylpropyl)carbaminsäure-benzylester,
{(S)-1-[(1S,2R,3R)-1-Benzyl-2-hydroxy-3-[(S)-1-(2-hydroxy-4-methoxybenzylcarbamoyl)-2-methylpropyl-carbamoyl]-3-(3,4,5-trimethoxybenzylamino)propylcarbamoyl]-2,2-dimethylpropyl}carbaminsäure-benzylester,
{(S)-1-[(1S,2R,3R)-1-Benzyl-2-hydroxy-3-[(S)-1-(2-hydroxy-4-methoxybenzylcarbamoyl)-2-methylpropyl-carbamoyl]-3-(2,3,4-trimethoxybenzylamino)propylcarbamoyl]-2,2-dimethylpropyl}carbaminsäure-benzylester,
((S)-1-{(1S,2R,3R)-1-Benzyl-3-(3,4-dimethoxybenzylamino)-2-hydroxy-3-[(S)-1-(2-hydroxy-4-methoxy-benzylcarbamoyl)-2-methylpropylcarbamoyl]propylcarbamoyl}-2,2-dimethylpropyl)carbaminsäure-benzylester,
((S)-1-{(1S,2R,3R)-1-Benzyl-3-(3,5-dimethoxybenzylamino)-2-hydroxy-3-[(S)-1-(2-hydroxy-4-methoxy-benzylcarbamoyl)-2-methylpropylcarbamoyl]propylcarbamoyl}-2,2-dimethylpropyl)carbaminsäure-benzylester,
{(S)-1-[(1S,2R,3R)-1-Benzyl-2-hydroxy-3-[(S)-1-(2-hydroxy-4-methoxybenzylcarbamoyl)-2-methylpropyl-carbamoyl]-3-(2,4,5-trimethoxybenzylamino)propylcarbamoyl]-2,2-dimethylpropyl}carbaminsäure-benzylester,
{(S)-1-[(1S,2R,3R)-1-Benzyl-2-hydroxy-3-[(S)-1-(2-hydroxy-4-methoxybenzylcarbamoyl)-2-methyl-propylcarbamoyl]-3-(3,4,5-trimethoxybenzylamino)propylcarbamoyl]-2,2-dimethylpropyl}-carbaminsäure-tert-butylester,
(2R,3R,4S)-4-[(S)-3,3-Dimethyl-2-(2-naphthalin-1-yl-acetylamino)butyrylamino]-3-hydroxy-5-phenyl-2-(3,4,5-trimethoxybenzylamino)pentansäure-[(S)-1-(2-hydroxy-4-methoxybenzylcarbamoyl)-2-methylpropyl]amid,
(2R,3R,4S)-4-[(S)-3,3-Dimethyl-2-(2-naphthalin-1-yl-acetylamino)butyrylamino]-3-hydroxy-5-phenyl-2-(2,3,4-trimethoxybenzylamino)pentansäure-[(S)-1-(2-hydroxy-4-methoxybenzylcarbamoyl)-2-methylpropyl]amid,
(2R,3R,4S)-4-[(S)-3,3-Dimethyl-2-(2-naphthalin-1-yl-acetylamino)butyrylamino]-3-hydroxy-5-phenyl-2-(2,4,5-trimethoxybenzylamino)pentansäure-[(S)-1-(2-hydroxy-4-methoxybenzylcarbamoyl)-2-methylpropyl]amid,
(2R,3R,4S)-4-[(S)-3,3-Dimethyl-2-(2-naphthalin-1-yl-acetylamino)butyrylamino]-3-hydroxy-2-[(naphthalin-1-ylmethyl)amino]-5-phenylpentansäure-[(S)-1-(2-hydroxy-4-methoxybenzylcarbamoyl)-2-methylpropyl]amid,
(2R,3R,4S)-2-(4-Dimethylaminobenzylamino)-4-[(S)-3,3-dimethyl-2-(2-naphthalin-1-yl-acetylamino)-butyrylamino]-3-hydroxy-5-phenylpentansäure-[(S)-1-(2-hydroxy-4-methoxybenzylcarbamoyl)-2-methylpropyl]amid,
und pharmazeutisch annehmbare Salze hiervon.

**8.** Pharmazeutisch annehmbares Salz einer Verbindung der Formel I nach einem der Ansprüche 1 bis 6.

**9.** Verbindung der Formel I nach einem der Ansprüche 1 bis 7 oder ein pharmazeutisch annehmbares Salz einer solchen Verbindung zur Verwendung in einem Verfahren zur therapeutischen Behandlung des menschlichen oder tierischen Körpers.

**10.** Pharmazeutische Zusammensetzung, die eine therapeutisch wirksame Menge einer Verbindung der Formel I nach

einem der Ansprüche 1 bis 7 oder ein pharmazeutisch annehmbares Salz einer solchen Verbindung zusammen mit einem pharmazeutischen Träger enthält.

**11.** Pharmazeutische Zusammensetzung zur Behandlung von proliferativen Erkrankungen bei Warmblütern, einschließlich dem Menschen, die eine Verbindung der Formel I nach einem der Ansprüche 1 bis 7 oder ein pharmazeutisch annehmbares Salz einer solchen Verbindung in einer Dosis, die gegen diese Erkrankung wirksam ist, zusammen mit einem pharmazeutischen Träger enthält.

**12.** Verwendung einer Verbindung der Formel I nach einem der Ansprüche 1 bis 7 oder eines pharmazeutisch annehmbaren Salzes einer solchen Verbindung zur Herstellung von pharmazeutischen Zusammensetzungen zur Verwendung bei der Behandlung von proliferativen Erkrankungen.

**13.** Verfahren zur Herstellung einer Verbindung der Formel I nach Anspruch 1 oder eines Salzes einer solchen Verbindung, **dadurch gekennzeichnet, dass**

a) eine Verbindung der Formel II

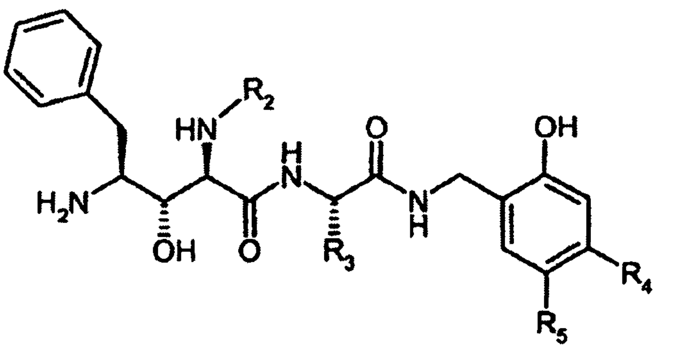

(II),

worin $R_2$, $R_3$, $R_4$ und $R_5$ wie für die Verbindungen der Formel I nach Anspruch 1 definiert sind, funktionelle Gruppen, die in einer Verbindung der Formel II vorkommen, mit Ausnahme der Gruppen, die an der Reaktion teilnehmen, geschützt sind, erforderlichenfalls durch leicht entfernbare Schutzgruppen oder als Salz einer solchen Verbindung, mit einer Säure der Formel III umgesetzt werden

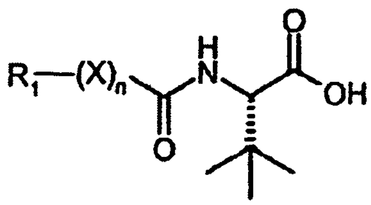

(III),

worin n, $R_1$ und X wie für Verbindungen der Formel I nach Anspruch 1 definiert sind, funktionelle Gruppen, die in einer Verbindung der Formel III vorkommen, mit Ausnahme der Gruppen, die an der Reaktion teilnehmen, geschützt sind, erforderlichenfalls durch leicht entfembare Schutzgruppen oder einem geeigneten reaktiven Säurederivat einer solchen Verbindung und alle vorhandenen Schutzgruppen entfernt werden

b) eine Verbindung der Formel IV

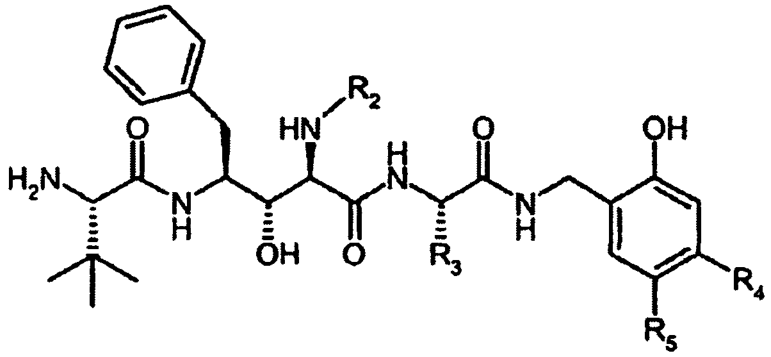

(IV),

worin $R_2$, $R_3$, $R_4$ und $R_5$ wie für die Verbindungen der Formel I nach Anspruch 1 definiert sind, funktionelle Gruppen, die in einer Verbindung der Formel IV vorkommen, mit Ausnahme der Gruppen, die an der Reaktion teilnehmen, geschützt sind, erforderlichenfalls durch leicht entfernbare Schutzgruppen, oder ein Salz einer solchen Verbindung, mit einer Säure der Formel V umgesetzt wird

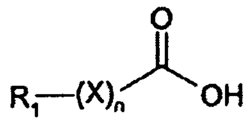

(V),

worin n, $R_1$ und X wie für Verbindungen der Formel 1 nach Anspruch 1 definiert sind, funktionelle Gruppen, die in einer Verbindung der Formel V vorkommen, mit Ausnahme der Gruppen, die an der Reaktion teilnehmen, geschützt sind, erforderlichenfalls durch leicht entfembare Schutzgruppen oder einem geeigneten reaktiven Säurederivat einer solchen Verbindung und alle vorhandenen Schutzgruppen abgespalten werden,
und erforderlichenfalls eine Verbindung der Formel I, die durch Verfahren a) oder b) erhalten wird, in eine andere Verbindung der Formel I umgesetzt wird, eine erhaltene freie Verbindung der Formel I in ein Salz umgewandelt wird oder ein erhaltenes Salz einer Verbindung der Formel I in ein anderes Salz oder in seine freie Form umgewandelt wird.

## Revendications

1. Composé de formule 1

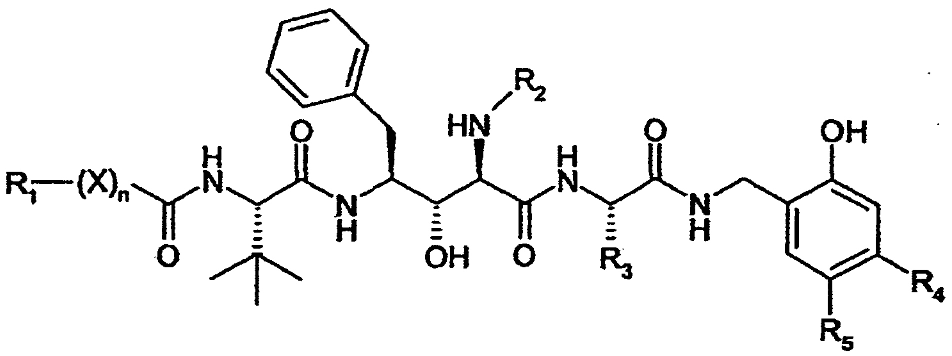

(I),

dans laquelle,
n est 0 ou 1 ;
$R_1$ et $R_2$, indépendamment l'un de l'autre, sont un radical aliphatique, ou un radical aromatique, aromatique-aliphatique, cycloaliphatique, cycloaliphatique-aliphatique, hétérocyclique ou hétérocyclique-aliphatique, chaque radical

ne possédant pas plus de 20 atomes de carbone ;
$R_3$ est hydrogène, oxa-alkyle, un radical aliphatique ou un radical possédant jusqu'à 20 atomes de carbone de formule $-(Y)_m-R_6$, dans laquelle Y est alkyle, m est 0 ou 1 et $R_6$ est un radical monocyclique non substitué ou substitué possédant 5 ou 6 membres cycliques contenant jusqu'à 3 hétéroatomes sélectionnés parmi le groupe composé d'azote, oxygène et soufre, **caractérisé en ce que** ledit radical monocyclique peut également être fusionné à un anneau benzo ;
$R_4$ et $R_5$, indépendamment l'un de l'autre, sont sélectionnés à partir du groupe composé d'hydrogène ; d'un radical aliphatique ; d'hydroxy libre éthérifié ou estérifié ; de carboxy libre ou estérifié ; de formyle ; d'alkanoyle ; d'amino non substitué, mono- ou di-substitué ; de mercapto ; de sulfo ; d'alkyl-thio ; de carbarnoyle ; de *N*-alkylcarbamoyle ; de *N,N*-di-alkyl-carbamoyle ; de cyano et de nitro ; **caractérisé en ce que** le carbone contenant les radicaux $R_4$ et $R_5$ possède jusqu'à 12 atomes de carbone ;
X est azote, oxygène ou soufre ; et
si l'un des radicaux $R_4$ ou $R_5$ est hydrogène, l'autre est tel que défini ci-dessus pour $R_4$ et $R_5$ à l'exception d'hydrogène, et si $R_4$ est méthoxy, $R_2$ est tel que défini ci-dessus à l'exception de benzyle monosubstitué en position 4 par halogène, alkyle, alcoxy ou hydroxyalcoxy ; ou un de ses sels.

**2.** Composé de formule I selon la revendication 1, dans laquelle
n est 0 ou 1 ;
$R_1$ ne possède pas plus de 20 atomes de carbone et est un membre sélectionné à partir du groupe composé de $C_1$-$C_7$ alkyle, cycloalkyl-$C_1$-$C_7$alkyle, phényl-$C_1$-$C_7$alkyle, naphtyl-$C_1$-$C_7$alkyle, et chinolyl-$C_1$-$C_7$ alkyle, les membres étant non substitués ou substitués par un ou plusieurs radicaux indépendamment sélectionnés à partir du groupe composé d'hydroxy, $C_1$-$C_7$alcoxy, $C_1$-$C_7$alkanoyloxy, halogène, carboxy, $C_1$-$C_7$alcoxycarbonyle, formyle, $C_1$-$C_7$alkanoyle, amino, *N*-$C_1$-$C_7$alkylamino, *N,N*-di- $C_1$-$C_7$alkylamino, mercapto, sulfo, $C_1$-$C_7$alkyl-thio, carbamoyle, *N*-$C_1$-$C_7$alkyl-carbamoyle, *N,N*-di- $C_1$-$C_7$alkyl-carbamoyle, cyano, nitro et $C_1$-$C_7$alkyle qui peut lui-même être substitué par lesdits radicaux ;
$R_2$ ne possède pas plus de 20 atomes de carbone et est un membre sélectionné à partir du groupe composé de phényl-$C_1$-$C_7$ alkyle, naphtyl-$C_1$-$C_7$alkyle, chinolyl- $C_1$-$C_7$alkyle, indolyl-$C_1$-$C_7$ alkyle et *N*-$C_1$-$C_7$ alkyl-indolyl-$C_1$-$C_7$alkyle, les membres étant non substitués ou substitués par un
ou plusieurs radicaux indépendamment sélectionnés à partir du groupe composé d'hydroxy, $C_1$-$C_7$alcoxy, $C_1$-$C_7$alkanoyloxy, halogène, carboxy, $C_1$-$C_7$alcoxycarbonyle, formyle, $C_1$-$C_7$alkanoyle, amino, *N*-$C_1$-$C_7$alkylamino, *N,N*-di-$C_1$-$C_7$alkylamino, mercapto, sulfo, $C_1$-$C_7$alkyl-thio, carbamoyle, *N*-$C_1$-$C_7$alkyl-carbamoyle, *N,N*-di-$C_1$-$C_7$alkyl-carbamoyle, cyano, nitro et $C_1$-$C_7$alkyle qui peut lui-même être substitué par lesdits radicaux ;
$R_3$ est hydrogène, un radical de formule $-G(-O-CH_2-CH_2)_t-R_7$, dans laquelle G et $R_7$ sont $C_1$-$C_7$alkyle et t est 1 à 3, $C_1$-$C_7$ alkyle non substitué
ou $C_1$-$C_7$ alkyle substitué par hydroxy, carboxy, amino, carbamoyle, guanidino, mercapto ou $C_1$-$C_7$alkyl-thio et ne possédant pas plus de 12 atomes de carbone, ou un radical de formule $-(Y)_m-R_6$, dans laquelle Y est $C_1$-$C_7$alkyle, m est 0 ou 1 et $R_6$ est phényle, hydroxy-phényle, imidazolyle, indolyle, pipéridyle, pipérazinyle, morpholinyle ou pyridyle ;
$R_4$ et $R_5$, indépendamment l'un de l'autre, sont sélectionnés à partir du groupe composé d'hydrogène, hydroxy, $C_1$-$C_7$alcoxy, $C_1$-$C_7$alkanoyloxy, halogène, carboxy, $C_1$-$C_7$alcoxycarbonyle, formyle, $C_1$-$C_7$alkanoyle, amino, *N*-$C_1$-$C_7$alkylamino, *N,N*-di-$C_1$-$C_7$alkylamino, mercapto, sulfo, $C_1$-$C_7$alkyl-thio, carbamoyle, *N*-$C_1$-$C_7$alkyl-carbamoyle, *N,N*-di-$C_1$-$C_7$alkyl-carbamoyle, cyano, nitro et $C_1$-$C_7$alkyle qui peut lui-même être substitué par lesdits radicaux, **caractérisé en ce que** le carbone contenant les radicaux $R_4$ et $R_5$ possède jusqu'à 12 atomes de carbone ; et
X est azote, oxygène ou soufre ; et
si l'un des radicaux $R_4$ ou $R_5$ est hydrogène, l'autre est tel que défini ci-dessus pour $R_4$ et $R_5$ à l'exception d'hydrogène et, si $R_4$ est méthoxy, $R_2$ est tel que défini ci-dessus à l'exception de benzyle monosubstitué en position 4 par halogène, alkyle, alcoxy ou hydroxyalcoxy ;
ou un de ses sels.

**3.** Composé de formule I selon la revendication 1, dans laquelle
n est 0 ou 1 ;
$R_1$ ne possède pas plus de 20 atomes de carbone et est un membre sélectionné à partir du groupe composé de $C_1$-$C_7$alkyle, cycloalkyl-$C_1$-$C_7$ alkyle, phényl-$C_1$-$C_7$alkyle, naphtyl-$C_1$-$C_7$alkyle et chinolyl-$C_1$-$C_7$alkyle, les membres étant non substitués ou substitués par un ou plusieurs radicaux indépendamment sélectionnés à partir du groupe composé d'un radical aliphatique ; d'hydroxy libre éthérifié ou estérifié ; de carboxy libre ou estérifié ; de formyle ; d'alkanoyle ; d'amino non substitué, mono- ou di-substitué ; de mercapto ; de sulfo ; d'alkyl-thio ; de carbamoyle ; de N-alkyl-carbamoyle ; de *N,N*-di-alkylcarbamoyle ; de cyano et de nitro ;
$R_2$ ne possède pas plus de 20 atomes de carbone et est un membre sélectionné à partir du groupe composé de

phényl-$C_1$-$C_7$alkyle, naphtyl-$C_1$-$C_7$alkyle, chinolyl-$C_1$-$C_7$alkyle, indolyl-$C_1$-$C_7$alkyle et *N*-$C_1$-$C_7$alkyl-indolyl-$C_1$-$C_7$alkyle, les membres étant non substitués ou substitués par un ou plusieurs radicaux indépendamment sélectionnés à partir du groupe composé d'un radical aliphatique ; d'hydroxy libre, éthérifié ou estérifié ; de carboxy libre ou estérifié ; de formyle ; d'alkanoyle ; d'amino non substitué, mono- ou di-substitué ; de mercapto ; de sulfo ; d'alkyl-thio ; de carbamoyle ; de *N*-alkyl-carbamoyle ; de *N,N*-di-alkyl-carbamoyle ; de cyano et de nitro ;
$R_3$ est hydrogène, oxa-alkyle, un radical aliphatique ou un radical possédant jusqu'à 20 atomes de carbone de formule -$(Y)_m$-$R_6$, dans laquelle Y est alkyle, m est 0 ou 1 et $R_6$ est un radical monocyclique non substitué ou substitué dotés de 5 ou 6 membres cycliques contenant jusqu'à 3 hétéroatomes sélectionnés à partir du groupe composé d'azote, oxygène et soufre, **caractérisé en ce que** ledit radical monocyclique peut également être fusionné à un anneau benzo ;
$R_4$ et $R_5$, indépendamment l'un de l'autre, sont sélectionnés à partir du groupe composé d'hydrogène ; d'un radical aliphatique ; d'hydroxy libre éthérifié ou estérifié ; de carboxy libre ou estérifié ; de formyle ; d'alkanoyle ; d'amino non substitué, mono- ou di-substitué ; de mercapto ; de sulfo ; d'alkyl-thio ; de carbamoyle ; de *N*-alkyl-carbamoyle ; de *N,N*-di-alkyl-carbamoyle ; de cyano et de nitro ; **caractérisé en ce que** le carbone contenant les radicaux $R_4$ et $R_5$ possède jusqu'à 12 atomes de carbone ; et
X est oxygène ; et
si l'un des radicaux $R_4$ ou $R_5$ est hydrogène, l'autre est tel que défini ci-dessus pour $R_4$ et $R_5$, à l'exception d'hydrogène et si $R_4$ est méthoxy, $R_2$ est tel que défini ci-dessus à l'exception benzyle monosubstitué en position 4 par halogène, alkyle, alcoxy ou hydroxyalcoxy ;
ou un de ses sels.

**4.** Composé de formule I selon la revendication 1, dans laquelle
n est 0 ou 1 ;
$R_1$ et $R_2$, indépendamment l'un de l'autre, sont un radical aliphatique, ou un radical aromatique, aromatique-aliphatique, cycloaliphatique, cycloaliphatique-aliphatique, hétérocyclique ou hétérocyclique-aliphatique, chaque radical ne possédant pas plus de 20 atomes de carbone ;
$R_3$ est hydrogène, un radical de formule -G(-O-$CH_2$-$CH_2$)$_t$-$R_7$, dans laquelle G et $R_7$ sont $C_1$-$C_7$alkyle et t est 1 à 3, $C_1$-$C_7$alkyle non substitué ou $C_1$-$C_7$alkyle substitué par hydroxy, carboxy, amino, carbamoyle, guanidino, mercapto ou $C_1$-$C_7$alkyl-thio et ne possédant pas plus de 12 atomes de carbone, ou un radical de formule -$(Y)_m$-$R_6$, dans laquelle Y est $C_1$-$C_7$alkyle, m est 0 ou 1 et $R_6$ est phényle, hydroxy-phényle, imidazolyle, indolyle, pipéridyle, pipérazinyle, morpholinyle ou pyridyle ;
$R_4$ et $R_5$, indépendamment l'un de l'autre, sont sélectionnés à partir du groupe composé d'hydrogène ; d'un radical aliphatique ; d'hydroxy libre, éthérifié ou estérifié ; de carboxy libre ou estérifié ; de formyle ; d'alkanoyle ; d'amino non substitué, mono- ou di-substitué ; de mercapto ; de sulfo ; d'alkyl-thio ; de carbamoyle ; de *N*-alkylcarbamoyle ; de *N,N*-di-alkyl-carbamoyle ; de cyano et de nitro ; **caractérisé en ce que** le carbone contenant les radicaux $R_4$ et $R_5$ possède jusqu'à 12 atomes de carbone ; et
X est oxygène ; et
si l'un des radicaux $R_4$ ou $R_5$ est hydrogène, l'autre est tel que défini ci-dessus pour $R_4$ et $R_5$, à l'exception d'hydrogène et si $R_4$ est méthoxy, $R_2$ est tel que défini ci-dessus à l'exception benzyle monosubstitué en position 4 par halogène, alkyle, alcoxy ou hydroxyalcoxy ;
ou un de ses sels.

**5.** Composé de formule I selon la revendication 1, dans laquelle
n est 0 ;
$R_1$ et $R_2$, indépendamment l'un de l'autre, sont un radical aliphatique, ou un radical aromatique, aromatique-aliphatique, cycloaliphatique, cycloaliphatique-aliphatique, hétérocyclique ou hétérocyclique-aliphatique, chaque radical ne possédant pas plus de 20 atomes de carbone ;
$R_3$ est hydrogène, oxa-alkyle, un radical aliphatique ou un radical possédant jusqu'à 20 atomes de carbone de formule -$(Y)_m$-$R_6$, dans laquelle Y est alkyle, m est 0 ou 1 et $R_6$ est un radical monocyclique non substitué ou substitué doté de 5 ou 6 membres cycliques contenant jusqu'à 3 hétéroatomes sélectionnés à partir du groupe composé d'azote, oxygène et soufre, **caractérisé en ce que** le radical monocyclique peut également être fusionné à un anneau benzo ; et
$R_4$ et $R_5$, indépendamment l'un de l'autre, sont sélectionnés à partir du groupe composé d'hydrogène ; d'un radical aliphatique ; d'hydroxy libre éthérifié ou estérifié ; de carboxy libre ou estérifié ; de formyle ; d'alkanoyle ; d'amino non substitué, mono- ou di-substitué ; de mercapto ; de sulfo ; d'alkyl-thio ; de carbamoyle ; de *N*-alkyl-carbamoyle ; de *N,N*-di-alkyl-carbamoyle ; de cyano et de nitro ; **caractérisé en ce que** le carbone contenant les radicaux $R_4$ et $R_5$ possèdent jusqu'à 12 atome de carbone ; et
si l'un des radicaux $R_4$ ou $R_5$ est hydrogène, l'autre est tel que défini ci-dessus pour $R_4$ et $R_5$, à l'exception d'hydrogène

et si $R_4$ est méthoxy, $R_2$ est tel que défini ci-dessus à l'exception benzyle monosubstitué en position 4 par halogène, alkyle, alcoxy ou hydroxyalcoxy ;
ou un de ses sels.

**6.** Composé de formule I selon la revendication 1, dans laquelle
n est 0 ou 1 ;
$R_1$ est $C_1$-$C_7$alkyle, phényl-$C_1$-$C_7$alkyle ou naphtyl-$C_1$-$C_7$alkyle ;
$R_2$ est mono-, di- ou tri-$C_1$-$C_7$alcoxy-phényl-$C_1$-$C_7$alkyle, *N,N*-di-$C_1$-$C_7$ alkyl-amino-phényl-$C_1$-$C_7$alkyle ou naphtyl-$C_1$-$C_7$alkyle ;
$R_3$ est $C_1$-$C_7$alkyle ;
$R_4$ et $R_5$, indépendamment l'un de l'autre, sont sélectionnés à partir du groupe composé d'hydrogène, $C_1$-$C_7$ alcoxy et halogène ;
X est oxygène ; et
si l'un des radicaux $R_4$ ou $R_5$ est hydrogène, l'autre est tel que défini ci-dessus pour $R_4$ et $R_5$, à l'exception d'hydrogène et si $R_4$ est méthoxy, $R_2$ est tel que défini ci-dessus à l'exception benzyle monosubstitué en position 4 par halogène, alkyle, alcoxy ou hydroxyalcoxy ;
ou un de ses sels.

**7.** Composé de formule I selon la revendication 1, sélectionné à partir du groupe composé de
ester benzylique d'acide ((S)-1-{(1S,2R,3R)-1-benzyl-2-hydroxy-3-[(S)-1 -(2-hydroxy-4-méthoxy-benzylcarbamoyl)-2-méthyl-propylcarbamoyl]-3-[(naphtalèn-1-ylméthyl)-amino]-propylcarbamoyl}-2,2-diméthyl-propyl)-carbamique,
ester benzylique d'acide ((S)-1-{(1S,2R,3R)-1-benzyl-3-(4-diméthylamino-benzylamino)-2-hydroxy-3-[(S)-1-(2-hydroxy-4-méthoxy-benzylcarbamoyl)-2-méthyl-propylcarbamoyl]-propylcarbamoyl}-2,2-diméthyl-propyl)-carbamique,
ester benzylique d'acide {(S)-1-[(1S,2R,3R)-1-benzyl-3-[(S)-1-(5-bromo-2-hydroxy-benzyl-carbamoyl)-2-méthyl-propylcarbamoyl]-2-hydroxy-3-(4-méthoxy-benzylamino)-propylcarbamoyl]-2,2-diméthyl-propyl}-carbamique,
ester benzylique d'acide {(S)-1-[(1S,2R,3R)-1-benzyl-2-hydroxy-3-[(S)-1 -(2-hydroxy-4-méthoxy-benzylcarbamoyl)-2-méthyl-propylcarbamoyl]-3-(3-méthoxy-benzylamino)-propylcarbamoyl]-2,2-diméthyl-propyl}-carbamique,
ester benzylique d'acide ((S)-1-{(1S,2R,3R)-1-benzyl-3-(2,4-diméthoxy-benzylamino)-2-hydroxy-3-[(S)-1-(2-hydroxy-4-méthoxy-benzylcarbamoyl)-2-méthyl-propylcarbamoyl)-propylcarbamoyl}-2,2-diméthyl-propyl)-carbamique,
ester benzylique d'acide {(S)-1-[(1S,2R,3R)-1-benzyl-2-hydroxy-3-[(S)-1-(2-hydroxy-4-méthoxy-benzylcarbamoyl)-2-méthyl-propylcarbamoyl]-3-(3,4,5-triméthoxy-benzylamino)-propylcarbamoyl]-2,2-diméthyl-propyl}-carbamique,
ester benzylique d'acide {(S)-1-[(1S,2R,3R)-1-benzyl-2-hydroxy-3-[(S)-1 -(2-hydroxy-4-méthoxy-benzylcarbamoyl)-2-méthyl-propylcarbamoyl]-3-(2,3,4-triméthoxy-benzylamino)-propylcarbamoyl]-2,2-diméthyl-propyl}-carbamique,
ester benzylique d'acide ((S)-1-{(1S,2R,3R)-1-benzyl-3-(3,4-diméthoxy-benzylamino)-2-hydroxy-3-[(S)-1-(2-hydroxy-4-méthoxy-benzylcarbamoyl)-2-méthyl-propylcarbamoyl]-propylcarbamoyl}-2,2-diméthyl-propyl)-carbamique,
ester benzylique d'acide ((S)-1-{(1S,2R,3R)-1-benzyl-3-(3,5-diméthoxy-benzylamino)-2-hydroxy-3-[(S)-1-(2-hydroxy-4-méthoxy-benzylcarbamoyl)-2-méthyl-propylcarbamoyl]-propylcarbamoyl)-2,2-diméthyl-propyl)-carbamique,
ester benzylique d'acide {(S)-1-[(1S,2R,3R)-1-benzyl-2-hydroxy-3-[(S)-1 -(2-hydroxy-4-méthoxy-benzylcarbamoyl)-2-méthyl-propylcarbamoyl]-3-(2,4,5-triméthoxy-benzylamino)-propylcarbamoyl]-2,2-diméthyl-propyl}-carbamique,
ester *tert*-butylique d'acide {(S)-1-((1S,2R,3R)-1-benzyl-2-hydroxy-3-[(S)-1-(2-hydroxy-4-méthoxy-benzylcarbamoyl)-2-méthyl-propylcarbamoyl]-3-(3,4,5-triméthoxy-benzylamino)-propylcarbamoyl]-2,2-diméthyl-propyl}-carbamique,
[(S)-1-(2-hydroxy-4-méthoxy-benzylcarbamoyl)-2-méthyl-propyl]-amide d'acide (2R,3R,4S)-4-[(S)-3,3-diméthyl-2-(2-naphtalen-1-yl-acétylamino)-butyrylamino]-3-hydroxy-5-phényl-2-(3,4,5-triméthoxy-benzylamino)-pentanoïque
[(S)-1-(2-hydroxy-4-méthoxy-benzylcarbamoyl)-2-méthyl-propyl]-amide d'acide (2R,3R,4S)-4-[(S)-3,3-diméthyl-2-(2-naphtalen-1-yl-acétylamino)-butyrylamino]-3-hydroxy-5-phényl-2-(2,3,4-triméthoxy-benzylamino)-pentanoïque
[(S)-1-(2-hydroxy-4-méthoxy-benzylcarbamoyl)-2-méthyl-propyl]-amide d'acide (2R,3R,4S)-4-[(S)-3,3-diméthyl-2-(2-naphtalen-1-yl-acétylamino)-butyrylamino]-3-hydroxy-5-phényl-2-(2,4,5-triméthoxy-benzylamino)-pentanoïque
[(S)-1-(2-hydroxy-4-méthoxy-benzylcarbamoyl)-2-méthyl-propyl]-amide d'acide (2R,3R,4S)-4-[(S)-3,3-diméthyl-2-(2-naphtalen-1-yl-acétylamino)-butyrylamino]-3-hydroxy-2-[(naphtalen-1-ylméthyl)-amino]-5-phényl-pentanoïque

[(S)-1-(2-hydroxy-4-méthoxy-benzylcarbamoyl)-2-méthyl-propyl]-amide d'acide (2R,3R,4S)-2-(4-diméthylamino-benzylamino)-4-[(S)-3,3-diméthyl-2-(2-naphtalen-1-yl-acétylamino)-butyrylamino]-3-hydroxy-5-phényl-pentanoïque,
et un de leurs sels pharmaceutiquement acceptables.

**8.** Sel pharmaceutiquement acceptable d'un composé de formule I selon l'une des revendications 1 à 6.

**9.** Composé de formule I selon l'une quelconque des revendications 1 à 7, ou un de ses sels pharmaceutiquement acceptables utilisable dans une méthode pour le traitement thérapeutique du corps humain ou animal.

**10.** Composition pharmaceutique comprenant une quantité thérapeutiquement efficace d'un composé de formule I selon l'une quelconque des revendications 1 à 7, ou d'un de ses sels pharmaceutiquement acceptables, en combinaison avec un véhicule pharmaceutique.

**11.** Composition pharmaceutique pour le traitement de maladies prolifératives affectant les animaux à sang chaud, y compris les êtres humains, comprenant, selon un dosage actif à l'encontre de ladite maladie, un composé de formule I selon l'une quelconque des revendications 1 à 7, ou d'un de ses sels pharmaceutiquement acceptables, en combinaison avec un véhicule pharmaceutique.

**12.** Utilisation d'un composé de formule I selon l'une quelconque des revendications 1 à 7, ou d'un de ses sels pharmaceutiquement acceptables, pour la préparation de compositions pharmaceutiques utilisables pour le traitement de maladies prolifératives.

**13.** Procédé de préparation d'un composé de formule I selon l'une quelconque des revendications 1 à 7, ou d'un de ses sels pharmaceutiquement acceptables, **caractérisé en ce que**

a) un composé de formule II

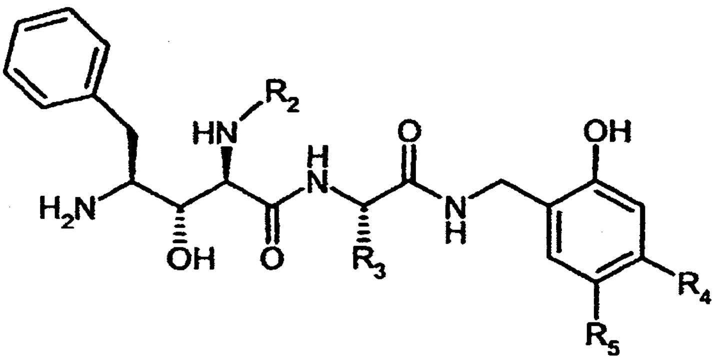

(II),

dans laquelle $R_2$, $R_3$, $R_4$ et $R_5$ sont tels que définis pour les composés de formule I selon la revendication 1, les groupes fonctionnels présents dans un composé de formule II, à l'exception des groupes participant à la réaction, étant protégés, si nécessaire, par des groupes protecteurs facilement éliminables, ou un de ses sels, est réagi avec un acide de formule III

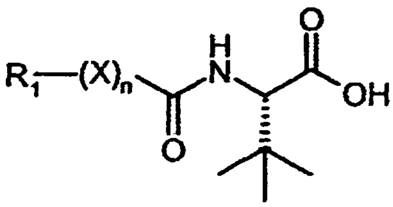

(III),

dans laquelle, n, $R_1$ et X sont tels que définis pour les composés de formule I selon la revendication 1, les groupes fonctionnels présents dans un composé de formule III, à l'exception des groupes participant à la réaction, étant protégés, si nécessaire, par des groupes protecteurs facilement éliminables, ou un de ses dérivés d'acide réactif adéquats, et tous les groupes protecteurs présents sont éliminés, ou

b) un composé de formule IV

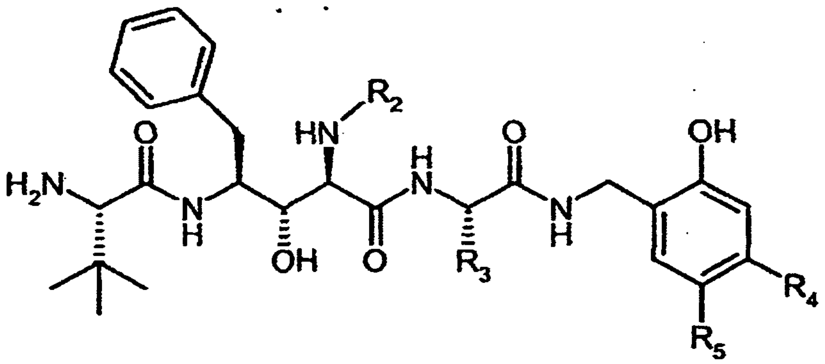

(IV),

dans laquelle $R_2$, $R_3$, $R_4$ et $R_5$ sont tels que définis pour les composés de formule I selon la revendication 1, les groupes fonctionnels présents dans un composé de formule IV, à l'exception des groupes participant à la réaction, étant protégés, si nécessaire, par des groupes protecteurs facilement éliminables, ou un de ses sels, est réagi avec un acide de formule V

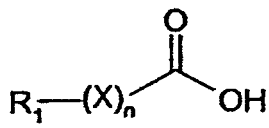

(V).

dans laquelle, n, $R_1$ et X sont tels que définis pour les composés de formule I selon la revendication 1, les groupes fonctionnels présents dans un composé de formule V, à l'exception des groupes participant à la réaction, étant protégés, si nécessaire, par des groupes protecteurs facilement éliminables, ou un de ses dérivés d'acide réactif adéquats, et tous les groupes protecteurs présents sont éliminés,

et, si souhaité, un composé de formule I obtenu à l'aide du procédé a) ou b) est converti en un autre composé de formule I, un composé libre obtenu de formule I est converti en un sel, ou sel obtenu d'un composé de formule I est converti en un autre sel ou en un de ses sels.